(19) Europäisches Patentamt
European Patent Office
Office européen des brevets

(11) **EP 3 215 007 B1**

(12) **EUROPÄISCHE PATENTSCHRIFT**

(45) Veröffentlichungstag und Bekanntmachung des Hinweises auf die Patenterteilung:
**04.09.2019 Patentblatt 2019/36**

(21) Anmeldenummer: **16704553.3**

(22) Anmeldetag: **04.02.2016**

(51) Int Cl.:
*A61B 5/0476* (2006.01)     *A61B 5/0488* (2006.01)
*A61B 5/053* (2006.01)      *A61B 5/11* (2006.01)
*A61F 7/00* (2006.01)       *A61N 1/36* (2006.01)
*A61N 5/06* (2006.01)       *A61B 5/00* (2006.01)
*A61B 5/0484* (2006.01)     *A61B 5/0478* (2006.01)
*A61N 1/04* (2006.01)       *A61B 5/04* (2006.01)
*A61H 23/00* (2006.01)      *A61H 23/02* (2006.01)
*A61F 7/02* (2006.01)       *G06Q 50/22* (2018.01)
*A61N 5/067* (2006.01)

(86) Internationale Anmeldenummer:
**PCT/EP2016/052389**

(87) Internationale Veröffentlichungsnummer:
**WO 2016/128291 (18.08.2016 Gazette 2016/33)**

(54) **VORRICHTUNG ZUR KALIBRIERUNG EINER NICHT-INVASIVEN MECHANISCH TAKTILEN UND/ODER THERMISCHEN NEUROSTIMULATION**

DEVICE FOR CALIBRATING A NON-INVASIVE MECHANICALLY TACTILE AND/OR THERMAL NEUROSTIMULATION

DISPOSITIF D'ÉTALONNAGE D'UNE NEUROSTIMULATION MÉCANIQUEMENT TACTILE ET/OU THERMIQUE NON INVASIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.02.2015 DE 102015101823**

(43) Veröffentlichungstag der Anmeldung:
**13.09.2017 Patentblatt 2017/37**

(73) Patentinhaber: **Forschungszentrum Jülich GmbH 52425 Jülich (DE)**

(72) Erfinder: **TASS, Peter Alexander 83684 Tegernsee (DE)**

(74) Vertreter: **Müller-Boré & Partner Patentanwälte PartG mbB Friedenheimer Brücke 21 80639 München (DE)**

(56) Entgegenhaltungen:
WO-A1-2011/127918     WO-A1-2013/117655
WO-A1-2013/135685

**Beschreibung**

Die Erfindung betrifft eine Vorrichtung zur Kalibrierung einer nicht-invasiven mechanisch taktilen und/oder thermischen Neurostimulation.

Bei Patienten mit neurologischen oder psychiatrischen Erkrankungen, z. B. Morbus Parkinson, essentiellem Tremor, Tinnitus, Dystonie oder Zwangserkrankungen, sind Nervenzellverbände in umschriebenen Bereichen des Gehirns krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Zur Behandlung derartiger Erkrankungen wurden Stimulationstechniken entwickelt, die gezielt krankhaft synchroner neuronaler Aktivität entgegenwirken. Die "Coordinated Reset" (CR)-Stimulation zeichnet sich hierbei durch große therapeutische Wirksamkeit und Sicherheit aus. Die nicht-invasive CR-Stimulation kann mit unterschiedlichen Reizmodalitäten realisiert werden: mittels sensorischer, z. B. vibrotaktiler Reize, mittels elektrischer oder magnetischer Reizung oder Ultraschallreizung peripherer Nerven oder mittels direkter elektrischer oder magnetischer Reizung oder Ultraschallreizung des Gehirns oder Rückenmarks.

Die mechanisch taktile, insbesondere vibrotaktile CR-Stimulation lässt sich z. B. zur Behandlung der Parkinsonschen Erkrankung anwenden. Weitere Indikationen stellen z. B. Epilepsien (mittels vibrotaktiler CR-Stimulation), Funktionsstörungen nach Schlaganfall (mittels vibrotaktiler CR-Stimulation), chronische Schmerzsyndrome (mittels vibrotaktiler und/oder thermischer CR-Stimulation), Migräne (z. B. mittels visueller CR-Stimulation) dar. Des Weiteren lassen sich diese Erkrankungen und andere Hirnerkrankungen mit transkranieller Magnetstimulation oder direkter elektrischer Stimulation des Gehirns oder direkter Hirnstimulation mittels Ultraschall behandeln.

Die nicht-invasiven CR-Stimulationsvorrichtungen und -verfahren sind deutlich nebenwirkungsärmer und kostengünstiger als invasive CR-Stimulationsvorrichtungen und -verfahren und sind damit für eine größere Anzahl von Patienten zugänglich.

Von zentraler Bedeutung für die Wirksamkeit der CR-Stimulation ist, dass (i) die applizierten Reize die zu stimulierende Neuronenpopulation auf invasive oder nicht-invasive Weise tatsächlich erreichen, d. h., dass nicht an falschen Stellen des Körpers gereizt wird, und (ii) dass diese Reizung an hinreichend verschiedenen Stellen des Körpers erfolgt, so dass hinreichend verschiedene neuronale Subpopulationen stimuliert werden. Bei invasiver CR-Stimulation wird die optimale Lokalisation der implantierten Elektrode im Rahmen der OP-Planung u. a. über detaillierte anatomische Informationen, z. B. aus kernspintomographischen Untersuchungen, sichergestellt. Bei den nicht-invasiven Stimulationsverfahren hingegen ist die Kalibrierung der Lokalisation der verschiedenen nicht-invasiven Aktoren, z. B. der Platzierung der mechanisch taktilen oder thermischen Stimulatoren auf der Haut in Relation zum betroffenen Körperteil, bis jetzt ein Problem.

In der Veröffentlichung der internationalen Patentanmeldung WO 2013/117655 A1 wird eine Vorrichtung zum Stimulieren von Neuronen mit einer pathologischen synchronen und oszillatorischen neuronalen Aktivität beschrieben, wobei die Vorrichtung eine nicht-invasive Stimulationseinheit zum Anlegen von Stimuli zur Stimulation der Neuronen eines Patienten umfasst.

Der Erfindung liegt die Aufgabe zugrunde, eine Vorrichtung anzugeben, die eine vom Untersucher unabhängige, automatisch durchgeführte, elektrophysiologisch basierte Kalibrierung der Stimulationsparameter ermöglichen. Insbesondere sollen geeignete Bereiche auf der Körperoberfläche des Patienten für die mechanisch taktile und/oder thermische Stimulation schnell und zuverlässig ermittelt werden, um geeignete Zielorte im Gehirn reizen zu können, so dass die CR-Stimulation effektiv ist. Ferner soll die Erfindung ermöglichen, (i) die Therapie wirksam durchzuführen, (ii) Nebenwirkungen zu vermeiden, (iii) die zur Parametereinstellung durchzuführende Untersuchung möglichst kurz, praktikabel und für den Patienten erträglich zu gestalten.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1         eine schematische Darstellung einer Vorrichtung zur mechanisch taktilen und/oder thermischen desynchronisierenden Neurostimulation während des Betriebs;

Fig. 2         ein Flussdiagramm zur Veranschaulichung der Kalibrierung der in Fig. 1 dargestellten Vorrichtung;

Fig. 3 und 4   schematische Darstellungen einer Manschette mit einem Array von Stimulationselementen und einer über die Manschette wandernden Aktivierungsgruppe;

Fig. 5         eine schematische Darstellung einer periodischen Abfolge von Reizen;

Fig. 6          eine schematische Darstellung eines Vibrationsreizes;

Fig. 7          eine schematische Darstellung einer Auswahl einer Aktivierungsgruppe mittels eines Tablets;

Fig. 8          eine schematische Darstellung einer CR-Stimulation mit zwei Aktivierungsgruppen;

Fig. 9          eine schematische Darstellung einer CR-Stimulation mit vier Aktivierungsgruppen;

Fig. 10         eine schematische Darstellung einer Ermittlung des optimalen Abstands zwischen zwei Aktivierungsgruppen;

Fig. 11         eine schematische Darstellung einer paarweisen Testung von Aktivierungsgruppen;

Fig. 12         eine schematische Darstellung einer iterativen Testung von Aktivierungsgruppen;

Fig. 13         eine schematische Darstellung einer Vorrichtung zur Kalibrierung einer mechanisch taktilen und/oder thermischen CR-Stimulation mit einer am Unterarm des Patienten fixierten Manschette; und

Fig. 14         eine schematische Darstellung einer Vorrichtung zur Kalibrierung einer mechanisch taktilen und/oder thermischen CR-Stimulation mit einer am Bauch des Patienten fixierten Manschette.

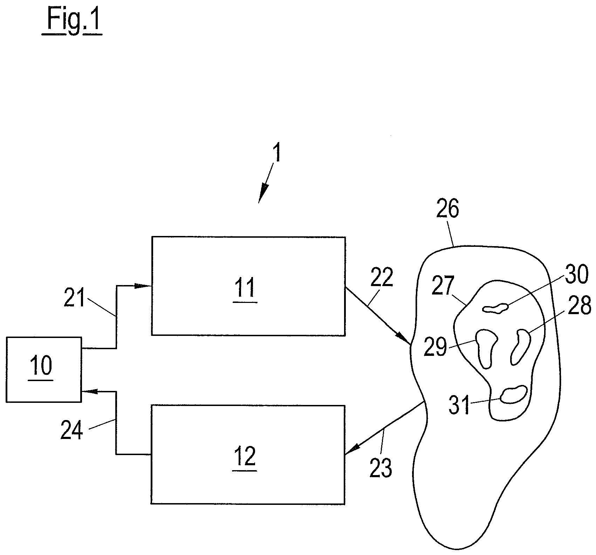

In Fig. 1 ist schematisch eine Vorrichtung 1 zur Stimulation von Neuronen mit einer krankhaft synchronen und oszillatorischen neuronalen Aktivität dargestellt. Mit Hilfe der Vorrichtung 1 lassen sich Stimulationsparameter einer nicht-invasiven Neurostimulation kalibrieren. Ferner kann die krankhaft synchrone und oszillatorische neuronale Aktivität unterdrückt und insbesondere desynchronisiert werden. Die Vorrichtung 1 besteht aus einer Steuer- und Analyseeinheit 10, einer Stimulationseinheit 11 und einer Messeinheit 12. Während des Betriebs der Vorrichtung 1 führt die Steuer- und Analyseeinheit 10 u. a. eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11, die insbesondere eine Mehrzahl von Stimulationselementen enthalten kann, erzeugt anhand der Steuersignale 21 Reize 22, die einem Patienten verabreicht werden. Die Reize 22 können mechanisch taktile Reize und/oder thermische Reize sein, wobei die mechanisch taktilen Reize insbesondere taktile, vibratorische, vibrotaktile oder über die Haut applizierte propriozeptive Reize sein können. Die Reize 22 können vom Patienten insbesondere bewusst wahrnehmbar sein. Die Stimulationseinheit 11 und insbesondere auch die Steuer- und Analyseeinheit 10 sind nicht-invasive Einheiten, d. h., während des Betriebs der Vorrichtung 1 befinden sie sich außerhalb des Körpers des Patienten und werden nicht operativ in den Körper des Patienten implantiert.

Der durch die Reize 22 erzielte Stimulationseffekt wird mit Hilfe der Messeinheit 12 kontrolliert. Die Messeinheit 12 nimmt ein oder mehrere am Patienten gemessene Messsignale 23 auf, wandelt diese gegebenenfalls in elektrische Signale 24 um und führt diese der Steuer- und Analyseeinheit 10 zu. Insbesondere kann mittels der Messeinheit 12 die pathologisch neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert (z. B. Muskelaktivität). Die Steuer- und Analyseeinheit 10 verarbeitet die Signale 24, z. B. können die Signale 24 verstärkt und gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 einen Prozessor, z. B. einen Mikrocontroller, enthalten. Die hierin beschriebenen Stimulationsverfahren können als Software-Code in einem der Steuer- und Analyseeinheit 10 zugeordneten Speicher abgelegt sein.

Die Messeinheit 12 enthält ein oder mehrere Sensoren, die Signale messen, die es insbesondere mit Hilfe einer adäquaten Datenanalyse ermöglichen, (a) ein "phase locking", d. h. eine Phasensynchronisation, zwischen einem (bzgl. des Timings) strikt periodischem Pulszug einerseits und der Phase der pathologischen oszillatorischen Aktivität andererseits und (b) eine Ab- bzw. Zunahme der Amplitude der pathologischen oszillatorischen Aktivität nachzuweisen. In einer Ausgestaltung ermöglichen es die Sensoren der Messeinheit 12 weiterhin, (c) eine Stimulus-induzierte Zurücksetzung der Phase der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren (SQUIDS). Die neuronale Aktivität kann auch indirekt durch Messung des Tremors oder durch Messung von Bewegungen mittels Akzelerometern oder Gyroskopen oder durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden.

Alternativ können die Sensoren in den Körper des Patienten implantiert sein. Als invasive Sensoren können beispielsweise epikortikale Elektroden, Tiefenhirnelektroden zur Messung von z. B. lokalen Feldpotentialen (LFP), sub- oder epidurale Hirnelektroden, subkutane EEG-Elektroden und sub- oder epidurale Rückenmarkselektroden dienen. Des Weiteren können an peripheren Nerven zu befestigende Elektroden als Sensoren eingesetzt werden.

Es kann durchaus vorgesehen sein, dass die einzelnen Komponenten der Vorrichtung 1, insbesondere die Steuer- und Analyseeinheit 10, die Stimulationseinheit 11 und/oder die Messeinheit 12, baulich voneinander getrennt sind. Die Vorrichtung 1 kann daher auch als System aufgefasst werden.

Die Vorrichtung 1 kann insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, chronischen Schmerzsyndromen, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer CR-Stimulation dargestellt. Im Gehirn 26 oder Rückenmark 26 des Patienten weist mindestens eine Neuronenpopulation 27 eine wie vorstehend beschriebene krankhaft synchrone und oszillatorische neuronale Aktivität auf. Die Stimulationseinheit 11 verabreicht dem Patienten die Reize 22 derart, dass die Reize 22 über Rezeptoren aufgenommen werden und von dort über das Nervensystem an die krankhaft aktive Neuronenpopulation 27 im Gehirn 26 und/oder Rückenmark 26 weitergeleitet werden. Die Reize 22 sind so ausgestaltet, dass die krankhaft synchrone Aktivität der Neuronenpopulation 27 als Ergebnis der Stimulation desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die von der Stimulationseinheit 11 applizierten mechanisch taktilen und/oder thermischen Reize 22 werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize 22 wirken. Die vibratorischen Reize 22 zielen vorwiegend auf die Tiefensensibilität ab. Die vibratorischen Reize 22 können von in der Haut, den Muskeln, dem Subkutangewebe und/oder den Sehnen des Patienten gelegenen Rezeptoren aufgenommen werden. Als Rezeptoren für die vibratorischen Reize 22 seien beispielhaft die Vater-Pacini-Körperchen genannt, die Vibrationsempfindungen und Beschleunigungen vermitteln. Die thermischen Reize 22 werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer.

Die gezielte Stimulation bestimmter Bereiche des Gehirns oder Rückenmarks wird durch die somatotope Zuordnung von Körperregionen zu diesen Bereichen ermöglicht. Aufgrund der somatotopen Gliederung der Nervenleitungsbahnen und zugehörigen Hirngebiete werden durch mechanisch taktile und/oder thermische Reize, die an unterschiedlichen Stellen der Körperoberfläche appliziert werden, unterschiedliche Neuronen stimuliert. Beispielsweise können die Stimulationselemente am Fuß, Unterschenkel und Oberschenkel oder aber an der Hand, dem Unterarm und Oberarm des Patienten angebracht werden, um dadurch bestimmte Neuronen zu stimulieren.

Die Stimulationseinheit 11 kann demnach unterschiedliche Bereiche des Gehirns 26 oder Rückenmarks 26 separat stimulieren, indem die applizierten Reize 22 über Nervenleitungen an unterschiedliche Zielgebiete, die im Gehirn 26 und/oder Rückenmark 26 liegen, weitergeleitet werden. Die Zielgebiete können während der Stimulation mit eventuell unterschiedlichen und/oder zeitversetzten Reizen 22 stimuliert werden.

Bei der CR-Stimulation werden der Neuronenpopulation 27, die eine krankhaft synchrone und oszillatorische neuronale Aktivität aufweist, Reize 22 verabreicht, welche in der Neuronenpopulation 27 ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 27 mittels einer gezielten Stimulation kontrolliert. Da es ferner möglich ist, die krankhafte Neuronenpopulation 27 an unterschiedlichen Stellen zu stimulieren, kann die Phase der neuronalen Aktivität der krankhaften Neuronenpopulation 27 an den unterschiedlichen Stimulationsstellen zu unterschiedlichen Zeitpunkten zurückgesetzt werden. Im Ergebnis wird dadurch die krankhafte Neuronenpopulation 27, deren Neuronen zuvor synchron und mit gleicher Frequenz und Phase aktiv waren, in mehrere Subpopulationen aufgespalten, die in Fig. 1 schematisch dargestellt sind und mit den Bezugszeichen 28, 29, 30 und 31 gekennzeichnet sind (beispielhaft sind hier vier Subpopulationen dargestellt). Innerhalb einer der Subpopulationen 28 bis 31 sind die Neuronen nach dem Zurücksetzen der Phase weiterhin synchron und feuern auch weiterhin mit derselben pathologischen Frequenz, aber jede der Subpopulationen 28 bis 31 weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen 28 bis 31 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 27 nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 27 in Subpopulationen 28 bis 31 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Im Folgenden wird eine Kalibrierung beschrieben, die mit der Vorrichtung 1 durchgeführt wird, um damit die optimalen Reizparameter für die nicht-invasive CR-Stimulation zu ermitteln.

Gemäß einer Ausgestaltung wird zunächst ein sogenannter Entrainment-Test durchgeführt. Dazu steuert die Steuer- und Analyseeinheit 10 die Stimulationseinheit 11 derart an, dass die Stimulationseinheit 11 zumindest einen Teil der Körperoberfläche des Patienten entlang eines Pfads abrastert und dabei periodisch Reize 22 appliziert, d. h., die periodischen Reize 22 sind nicht stationär, sondern "wandern" während der Abrasterung über die Körperoberfläche. Anhand der in Reaktion auf die periodische Applikation der Reize 22 aufgenommenen Messsignale 23 wählt die Steuer- und Analyseeinheit 10 mindestens zwei Bereiche auf der Körperoberfläche des Patienten entlang des Pfads aus, in welchen die Phasensynchronisation zwischen der periodischen Applikation der Reize 22 und der neuronalen Aktivität der stimulierten Neuronen jeweils ein lokales Maximum aufweist. Anschließend wird an den mindestens zwei ausgewählten Bereichen eine CR-Stimulation durchgeführt. Dazu steuert die Steuer- und Analyseeinheit 10 die Stimulationseinheit 11 derart an, dass diese in den mindestens zwei ausgewählten Bereichen Reize 22 appliziert, die eine Phasenrücksetzung der neuronalen Aktivität der stimulierten Neuronen bewirken. Die in den mindestens zwei Bereichen applizierten Reize 22 sind gegeneinander zeitversetzt.

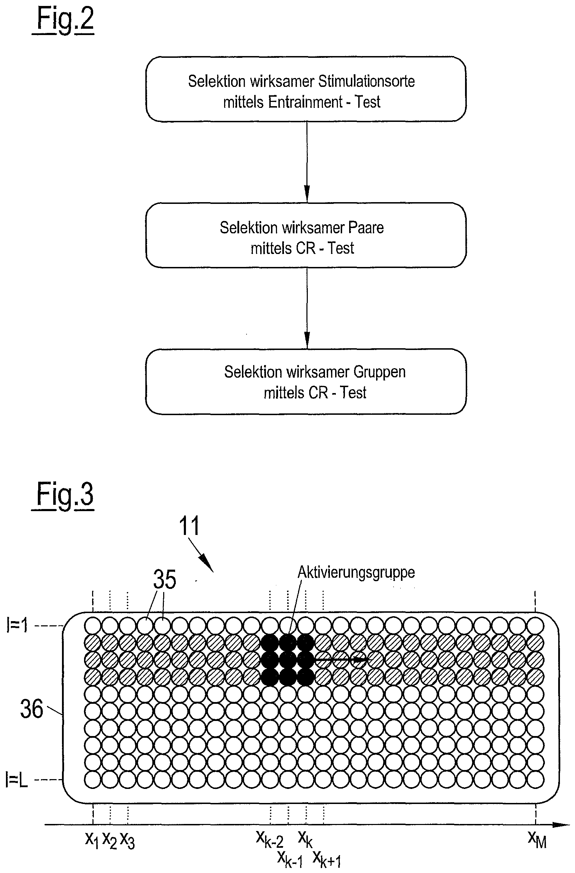

Die vorstehende Ausgestaltung kann weitergebildet werden, indem der Kalibrierung weitere Schritte hinzugefügt werden, die im Flussdiagramm von Fig. 2 zusammengefasst sind. Dazu wird in einem ersten Schritt der vorstehend beschriebene Entrainment-Test durchgeführt. Danach wird in einem zweiten Schritt ein Paar-Test vorgenommen, bei dem Reize an zwei verschiedenen Orten appliziert werden, um ein oder mehrere wirksame Paare für die CR-Stimulation zu selektieren. Schließlich wird in einem dritten Schritt ein Gruppen-Test durchgeführt, der die Applikation von Reizen an mindestens zwei und typischerweise mehr als zwei verschiedenen Orten vorsieht.

Die Vorrichtung 1 ermöglicht eine vom Untersucher unabhängige, automatisch durchgeführte, elektrophysiologisch basierte Kalibrierung der Stimulationsparameter, die im Vergleich zu herkömmlichen Kalibrierungen mit deutlich geringeren Reizstärken auskommt. Das hier genutzte Phasen-Entrainment ist ein dynamischer Vorgang, der bei sehr geringen Reizstärken effektiv ist. Schwächere Reize haben den Vorteil, dass die mit diesen Reizen durchgeführte Kalibrierung deutlich genauer ist. Der Grund hierfür ist, dass die schwächeren Reize anatomisch selektiver sind, da sie weniger Neuronen erreichen, nämlich nur die, welche optimal zu dem applizierten Reiz passen (und nicht benachbarte Neuronenpopulationen im Gehirn, die bei steigender Reizstärke mit stimuliert werden). Die Kalibrierung wird bei einer Reizintensität durchgeführt, bei der auch die Therapie tatsächlich durchgeführt wird. Schwächere Reize sind darüber hinaus für Patienten, insbesondere Schmerzpatienten weniger belastend. Beispielsweise können schon leichtere Berührungen oder Wärmereize von Patienten mit chronischen Schmerzsyndromen, z. B. mit Morbus Sudeck oder Neuralgien, als unangenehm oder sogar schmerzhaft empfunden werden.

Darüber hinaus ist die mit der Vorrichtung 1 durchgeführte Kalibrierung im Vergleich zu herkömmlichen Kalibrierungen deutlich schneller. Dies beugt einer Ermüdung des Patienten und der damit verbundenen Verschlechterung der Qualität der Daten, d. h. der Ergebnisse vor. Sie ist dadurch für den Patienten deutlich angenehmer, erfordert keine unzumutbare Compliance (Kooperativität der Patienten) und ist in der Praxis gut anwendbar.

Zusammenfassend ermöglicht es die Vorrichtung 1, (i) die Therapie wirksam durchzuführen, (ii) Nebenwirkungen zu vermeiden und (iii) die zur Parametereinstellung durchzuführenden Untersuchungen möglichst kurz, praktikabel und für den Patienten erträglich zu gestalten.

Im Folgenden werden die einzelnen zur Kalibrierung der Stimulationsparameter durchzuführenden Schritte detaillierter erläutert. Dies geschieht anhand eines Ausführungsbeispiels der Stimulationseinheit 11, welches in Fig. 3 schematisch dargestellt ist. Die Stimulationseinheit 11 umfasst hier ein Array von Stimulationselementen 35. Jedes der Stimulationselemente 35 kann mechanisch taktile und/oder thermische Reize erzeugen. Die Stimulationselemente 35 sind in dem vorliegenden Ausführungsbeispiel Bestandteil einer Manschette 36, die zur Stimulation an einem Körperteil des Patienten, beispielsweise einem Arm, befestigt werden kann. In Fig. 3 stehen l und m für die Reihen bzw. Spalten des Arrays aus Stimulationselementen 35, wobei $l = 1,..., L$ und $m = 1,..., M$ gilt. Insgesamt besteht das Array folglich aus LxM Stimulationselementen 35. Jedes der Stimulationselemente 35 kann von der Steuer- und Analyseeinheit 10 individuell angesteuert werden.

Die Manschette 36 mit dem Array aus den Stimulationselementen 35 ermöglicht es, periodisch Reize zu applizieren, wobei die Stellen, an denen die Reize appliziert werden, sich kontinuierlich ändern können. Auf diese Weise können größere Teile der Körperoberfläche abgerastert werden, so dass mittels Analyse der reizinduzierten neuronalen Aktivität geeignete Bereiche und Abstände zwischen diesen Bereichen für die therapeutische Mehrkanal-Stimulation ermittelt werden können.

Hierfür könnten prinzipiell natürlich auch entsprechende Aktoren über die Körperoberfläche gleiten. Einfacher ist es jedoch, größere Arrays von Stimulationselementen 35 z. B. mittels der Manschette 36 am Körper des Patienten zu fixieren und die aktivierten Gruppen von Stimulationselementen 35 zeitlich zu variieren. Z. B. können die Stimulationselemente 35 von der Steuer- und Analyseeinheit 10 so angesteuert werden, dass die Aktivierung der Stimulationselemente 35 z. B. entlang der Manschette 36 wandert.

Beispielhaft ist in Fig. 3 die wandernde Aktivierung einer 3x3-Gruppe von Stimulationselementen 35 gezeigt. Die Stimulationselemente 35 in der zweit- bis viertobersten Zeile, die durch eine Schraffur hervorgehoben sind, bilden sozusagen den Pfad, entlang dem die Aktivierung der Stimulationselemente 35 wandert. Die Bewegungsrichtung ist durch einen Pfeil dargestellt. Die zu einem bestimmten Zeitpunkt aktivierten Stimulationselemente 35 bilden eine 3x3-Gruppe und sind schwarz dargestellt. Die Aktivierungsgruppe entspricht einem Bereich auf der Körperoberfläche des Patienten, der von den von der Aktivierungsgruppe umfassten Stimulationselementen 35 stimuliert wird. Nur Stimulationselemente 35, die zumindest teilweise innerhalb der 3x3-Form der Aktivierungsgruppe liegen, erzeugen in dem vorliegenden Ausführungsbeispiel Reize. Alle übrigen Stimulationselemente 35, d. h., diejenigen Stimulationselemente, die vollständig außerhalb der 3x3-Form der Aktivierungsgruppe liegen, erzeugen keine Reize. Wenn die Aktivierungsgruppe über die Manschette 36 wandert, wandert der Bereich, in dem mechanisch taktile und/oder thermische Reize erzeugt werden, in entsprechender Weise über die Körperoberfläche des Patienten.

Wir bezeichnen die Position der Front der wandernden 3x3-Aktivierungsgruppe mit $X_F$. Die Front und damit die gesamte 3x3-Aktivierung kann z. B. mit konstanter Geschwindigkeit vom linken Ende der Manschette 36 zum rechten Ende der Manschette 36 wandern. In Fig. 3 befindet sich die Front bei $x_k$. Im Folgenden soll die Erfindung beispielhaft anhand der Realisation mit vibrotaktilen Stimulationselementen 36 erklärt werden. In diesem Fall ist jedes Stimulationselement 36 ein jeweiliger Aktor, der auf der Körperoberfläche angebracht ist und aus seiner Ruhelage ausgelenkt werden kann, um in die Körperoberfläche einzudrücken und dadurch den gewünschten Reiz zu erzeugen.

Die Amplitude der zur vibrotaktilen Stimulation eingesetzten Stimulationselemente 36, d. h. der Aktoren, zur Zeit t wird mit $A_{i,m}(t)$ bezeichnet. Hiermit lautet die Amplitude der Vibration der Stimulationselemente 36 in der k-ten Spalte, d. h. bei $x_k$, in der zweit- bis viertobersten Zeile folgendermaßen:

Für $X_F(t) \leq x_{k-1}$:  $\qquad A_{l,k}(t) = 0$

Für $x_{k-1} < X_F(t) \leq x_k$:

$$A_{l,k}(t) = A_{\max} \frac{X_F(t) - x_{k-1}}{x_k - x_{k-1}}$$

Für $x_k \leq X_F(t) \leq x_{k+3}$:  $\qquad A_{l,k}(t) = A_{\max}$

(fortgesetzt)

Für $x_{k+3} < X_F(t) < x_{k+4}$:

$$A_{l,k}(t) = A_{max}\left(1 - \frac{X_F(t) - x_{k+3}}{x_{k+4} - x_{k+3}}\right)$$

Für $X_F(t) \geq x_{k+4}$:  $\qquad A_{l,k}(t) = 0$

wobei l = 2, 3, 4 gilt.

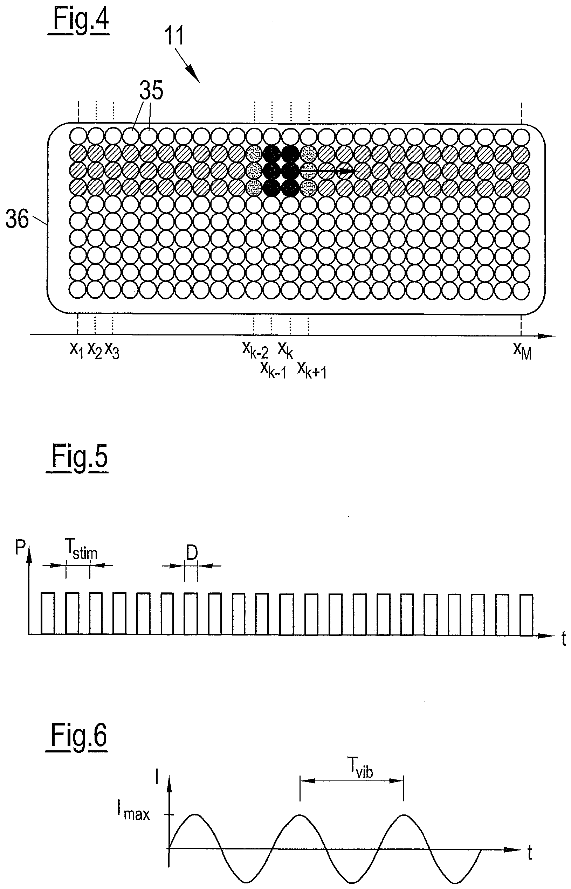

In Fig. 4 ist die Aktivierung einer 3x3-Gruppe von Stimulationselementen 36 im Vergleich zur Fig. 3 bereits einen halben Schritt weiter nach rechts gewandert. Durch Interpolation ist die Vibrationsamplitude der rechten und linken vertikalen Dreiergruppe der wandernden Aktivierung nicht maximal (dunkelgrau dargestellt).

Neben der hier verwandten linearen Interpolation können auch nichtlineare Interpolationsverfahren verwandt werden. Statt der Front $X_F(t)$ kann man analog z. B. auch die Position des Schwerpunkts der Gruppen-Aktivierung betrachten, welche in Fig. 3 bei $x_{k-1}$ liegt.

Während die Aktivierungsfront $X_F(t)$ mit konstanter Geschwindigkeit langsam entlang der Manschette 36 von links nach rechts, z. B. vom Handgelenk zur Ellenbeuge, wandert, wird eine periodische Abfolge P(t) von Reizen appliziert. Die periodische Abfolge P(t) ist schematisch in Fig. 5 dargestellt. Typischerweise handelt es sich, u. a. wegen der Trägheit der Aktoren, bei dieser Abfolge um eine glattere, nicht rechteckförmige Abfolge. Um den Zeitgang der Vibrationsamplitude eines konkreten Aktors zu bestimmen, wird das Produkt aus globaler periodischer Abfolge P(t) und lokaler Amplitude Ai,m(t), also P(t)Ai,m(t) berechnet. Das gesamte Vibrationssignal eines konkreten Aktors lautet folglich P(t)Ai,m(t)V(t), wobei V(t) eine hochfrequente Vibration, also z. B. eine Sinus- oder Rechteckschwingung mit einer Frequenz von beispielsweise 250 Hz ist. Es kann sich bei V(t) auch um eine niederfrequente Sinus- oder Rechteckschwingung im Bereich von 0,25 bis 50 Hz handeln. In einer weiteren Ausgestaltung können die Aktoren (bzw. Vibratoren) - getriggert durch P(t) - auch in die Haut gedrückt werden, so dass die auf diese Weise ausgelenkten Aktoren nicht um die Nulllinie oszillieren (vibrieren), sondern um die Auslenkung Po. In diesem Fall lautet das gesamte Vibrationssignal z. B. P(t)[Ai,m(t)V(t) + Po].

$A_{l,m}(t)$ gibt folglich die Amplitude jedes Aktors (bei einem jeweiligen l, m) zu einem gegebenen Zeitpunkt t an. Ferner gibt Ai,m(t) den Ort an, an dem sich die in Fig. 3 beispielhaft dargestellte aktivierte Gruppe von Aktoren zu dem jeweiligen Zeitpunkt t befindet.

P(t) besteht aus einer periodischen Abfolge von Pulsen. Die Pulse bestimmen die Zeiträume, zu denen Reize appliziert werden. Während der Zeiträume zwischen den Pulsen, wenn P(t) gleich Null ist, werden keine Reize appliziert. Wie Fig. 5 zeigt, weist die periodische Abfolge P(t) die Periode $T_{stim}$ auf. Die Stimulationsfrequenz $f_{stim} = 1/T_{stim}$ liegt typischerweise im Bereich von 0,25 bis 30 Hz. Die Dauer $D_{stim}$ der Pulse, d. h. die Dauer eines Stimulationsintervalls, kann im Bereich von 10 bis 1000 ms liegen. Die Pulse der periodischen Abfolge P(t) können insbesondere identisch sein.

Die Periode $T_{stim}$ wird nahe bei der mittleren Periode der pathologischen Oszillation gewählt. Die Stimulationsfrequenz $f_{stim} = 1/T_{stim}$ wird gemäß dem Fachmann bekannten Vorwissen bzgl. der für die jeweilige Erkrankung charakteristischen pathologischen Frequenzbänder (also in Übereinstimmung mit den pathologischen Rhythmen, die mit der CR-Stimulation desynchronisiert werden sollen) gewählt oder mittels Feedback durch Messung der zu desynchronisierenden pathologischen neuronalen Aktivität über Sensoren und Bestimmung des Frequenzpeaks im dem Fachmann bekannten pathologischen Frequenzband angepasst. Ferner kann ein Literaturwert für die mittlere Periode der pathologischen Oszillation herangezogen werden, und die für die Stimulation verwendete Periode $T_{stim}$ kann von diesem Literaturwert um z. B. bis zu $\pm$ 5%, $\pm$ 10% oder $\pm$ 20% abweichen.

Fig. 6 zeigt als Beispiel für die Vibration V(t) eine kontinuierliche Sinusschwingung mit einer Frequenz $f_{vib} = 1/T_{vib}$ im Bereich von 0,25 bis 300 Hz, insbesondere im Bereich von 0,25 bis 50 Hz oder 100 bis 300 Hz, wobei $T_{vib}$ die Periodendauer des Vibrationsreizes ist. Anstelle einer Sinusform können die vibratorischen Reize auch eine andere Form aufweisen, z. B. eine Rechteckform. Die Auslenkung $l_{max}$ eines jeweiligen Stimulationselements 35 zur Erzeugung der Vibration V(t) kann im Bereich von 0,1 bis 0,5 mm oder bis zu 5 mm liegen. Bei einer Frequenz $f_{vib}$ von 300 Hz kann ein Stimulationselement 35 eine Kraft von z. B. etwa 2 N auf die Körperoberfläche des Patienten ausüben.

Für den Fall, dass thermische Reize von den Stimulationselementen 35 appliziert werden sollen, kann jedes Stimulationselement 35 derart ausgestalten sein, dass es auf der Körperoberfläche des Patienten oder in einem tieferliegenden Bereich eine vorgegebene Temperatur $T_{temp}$ erzeugen kann. Die Temperatur $T_{temp}$ kann ober- oder unterhalb der ohne die Applikation des thermischen Reizes im Zielgebiet herrschenden Temperatur To liegen, d. h., das Zielgebiet kann erhitzt oder gekühlt werden. Die Temperatur $T_{temp}$ kann z. B. im Bereich von 22 bis 42 °C liegen. Die Temperatur $T_0$ ist in der Regel die Körpertemperatur des Patienten. In einer Ausgestaltung handelt es sich bei den Stimulationselementen 35 um die Ausgänge von Lichtleitern, die insbesondere von einem gemeinsamen Laser gespeist werden. Das Laserlicht kann mit einer geeigneten Steuerung derart auf die Lichtleiter verteilt werden, dass die gewünschte Applikation thermischer Reize erzielt wird.

Das gesamte Thermosignal eines Stimulationselements 35 berechnet sich aus $P(t)H_{l,m}(t)$, wobei $P(t)$ die periodische Abfolge von Pulsen ist, wie sie vorstehend im Zusammenhang mit Fig. 5 erläutert wurde, und $H_{l,m}(t)$ analog zu $A_{l,m}(t)$ gebildet ist und die von jedem Stimulationselement 35 (bei einem jeweiligen l, m) zu einem gegebenen Zeitpunkt t erzeugte Temperatur angibt. Insbesondere wird durch $H_{l,m}(t)$ der Ort angegeben, an dem sich die aktivierte Gruppe von Stimulationselementen 35 zu dem jeweiligen Zeitpunkt t befindet.

Während die Aktivierung der Stimulationselemente 35 z. B. von links nach rechts über die Manschette 36 entlang eines Pfads wandert, wird das Phasen-Entrainment zwischen $P(t)$ und der krankhaft rhythmischen neuronalen Aktivität in einem gleitenden Fenster berechnet. Dabei werden Bereiche auf der Körperoberfläche des Patienten entlang des Pfads identifiziert, in denen die Phasensynchronisation zwischen $P(t)$ und der krankhaft rhythmischen neuronalen Aktivität der stimulierten Neuronen jeweils ein lokales Maximum aufweist. Die Fensterlänge sollte so gewählt werden, dass sie mindestens 10, besser 50 oder sogar 100 mittleren Perioden des krankhaften neuronalen Rhythmus entspricht. Hieraus ergibt sich die Bewegungsgeschwindigkeit der Aktivierungsfront $X_F(t)$. Mit wachsender Fensterlänge wächst die räumliche Auflösung, aber auch die Untersuchungszeit. In der Praxis hat sich auch ein mehrschrittiges Vorgehen bewährt: zuerst mit einer kleineren Fensterlänge große Körperpartien abrastern, um Körperstellen mit gutem Phasen-Entrainment grob zu detektieren, um danach nur diese besonderen Körperstellen mit größerer Fensterlänge detailliert abzurastern.

Der Startpunkt der aktivierten Gruppe von Stimulationselementen 35, dies ist der linke Rand der Manschette 36 in den Beispielen von Fig. 3 und 4, sowie der Weg, welchen die Aktivierung zurücklegt, können unterschiedliche Formen annehmen und auf unterschiedliche Weise gewählt werden. Die Form der Aktivierungsgruppe, d. h. die 3x3-Gruppe in den Beispielen von Fig. 3 und 4, kann vorgegeben sein oder kann durch den Anwender, d. h. einen Arzt, medizinisches Fachpersonal oder den Patienten, eingestellt werden, z. B. über entsprechende Schalter. Beispielsweise können jedem Stimulationselement 35 oder kleineren Gruppen von Stimulationselementen 35 Druckschalter in der Manschette 36 zugeordnet sein oder aber der Anwender kann über ein Tablet, z. B. ein iPad, oder dergleichen die Form der Aktivierungsgruppe einstellen.

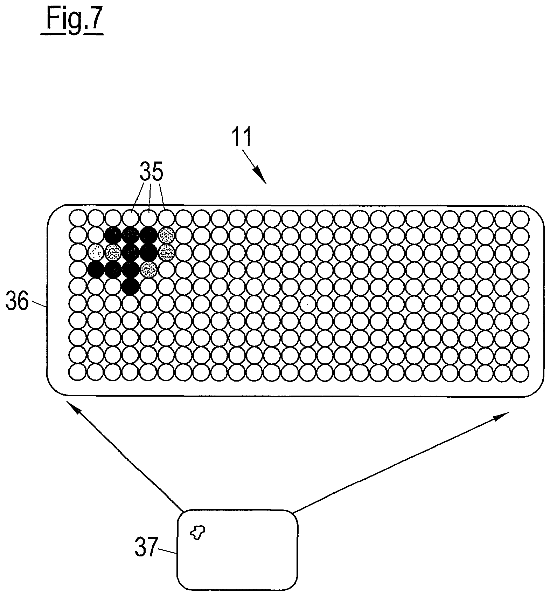

Fig. 7 zeigt schematisch ein Tablet 37, z. B. ein iPad, über welches die Startkonfiguration der Aktivierungsgruppe eingestellt wird. Hierzu wird an dem Tablet 37 eine entsprechende Gruppe, z. B. mittels eines Touchscreens, markiert. Optional können die zugehörigen Stimulationselemente 35 hierdurch aktiviert werden, wodurch z. B. durch die Rückmeldung des Patienten ("Wo spüren Sie die Aktivierung?") oder ein (nur während dieser Testphase stattfindendes) Aufblinken der zu den entsprechenden Stimulationselementen 35 gehörenden LEDs auf der Manschette 36 der Anwender sieht, wo die Aktivierung erfolgt. Das Tablet 37 ist vorzugsweise schnurlos mit der Manschette 36 verbunden. Letztere ist mit einer Batterie und optional auch mit einer Steuereinheit für den Patienten verbunden. Die Steuereinheit erlaubt es dem Patienten, die Manschette 36 auf einfache Weise zu bedienen, ohne extra das Tablet 37 verwenden zu müssen. Batterie und Steuereinheit können mit der Manschette 36 auch baulich vereint sein.

Die Form der Aktivierungsgruppe kann gemäß einer Ausgestaltung langsam variiert werden. Z. B. kann die Form der Aktivierungsgruppe langsam variiert werden, indem vom Schwerpunkt ausgehend die Aktivierungsgruppe zentrisch um z% gestreckt wird, wobei z langsam vom Ausgangswert (100%) bis zu einem Maximalwert, z. B. 110% bei z = 10, variiert wird. Analog zu der Variation der Position der Aktivierungsfront wird für die Phasen-Entrainment-Analyse statt der Position der Aktivierungsfront in diesem Fall der Wert der Streckung z herangezogen. Auf diese Weise können Konfigurationen der Aktivierungsgruppe an die individuelle Anatomie und Physiologie des Patienten, insbesondere die Größe der rezeptiven Felder, automatisch angepasst und damit optimiert werden.

Der Weg der Aktivierungsgruppe kann vorgegeben oder zufällig gewählt sein oder vom Anwender z. B. mittels Druckknöpfen in der Manschette 36 oder mit Hilfe des Tablets 37 eingestellt werden. Hierzu kann der Anwender z. B. auf dem Tablet 37 mehrere anatomisch bzw. physiologisch bevorzugte Zielpositionen vorgeben. Anschließend fährt die Aktivierungsgruppe den durch diese Orte vorgegebenen und mittels den üblichen Interpolationsverfahren ermittelten Weg ab.

Die Auswahl der Orte bzw. Bereiche, an denen die für die Therapie des Patienten vorgesehenen Reize appliziert werden, erfolgt mit Hilfe eines Entrainment-Tests. Der Entrainment-Test hat den Vorteil gegenüber anderen Tests, dass er deutlich schneller und sensitiver ist. Außerdem ermöglicht die geringe Reizstärke beim Entrainment-Test eine größere anatomische Genauigkeit, da kleinere Neuronenpopulationen gereizt werden. Dies ist für die Kalibrierung der CR-Stimulation wichtig, da hierbei über die einzelnen Stimulationskanäle möglichst separate, im Idealfall sogar disjunkte Subpopulationen gereizt werden sollen.

Der Entrainment-Test beruht auf der Ermittlung der Ausprägung einer Phasensynchronisation zwischen einem periodischen Reiz und dem neuronalen Rhythmus der krankhaft synchronen und oszillatorischen Neuronenpopulation. Die periodische Abfolge $P(t)$, die beispielhaft in Fig. 5 dargestellt ist, appliziert zu den Zeiten $\tau_1, \tau_2,..., \tau_K$ einen Reiz, wobei K die Anzahl der Reize bezeichnet. Da die Reizabfolge periodisch ist, ist die Stimulationsperiode $T_{stim}$ konstant, d. h., es gilt $T_{stim} = \tau_{j+i} - \tau_j$ für alle j = 1,..., K-1. Die zugehörige Stimulationsfrequenz, d. h. Repetitionsrate, lautet $f_{stim} = 1/T_{stim}$.

Die Phase der periodischen Reizabfolge P(t) lautet $\varphi_1(t) = 2\pi(t - \tau_1)/T_{stim}$ (vgl. z. B. M. G. Rosenblum, A. S. Pikovsky, C. Schäfer, J. Kurths, P. A. Tass: Phase Synchronization: From Theory to Data Analysis. In: Moss F. (Ed.): Handbook of Biological Physics, Elsevier (2000)). Die so gewählte Phase verschwindet, wenn der erste Reiz appliziert wird: $\varphi_1(t) = 0$.

Alternativ kann auch eine Phasenverschiebung "eingebaut" werden, die nichts an den Ergebnissen verändert, und auch keine Vorteile mit sich bringt. Eine Phasenverschiebung kann dadurch "eingebaut" werden, dass entweder eine andere Startzeit gewählt wird $\varphi_1(t) = 2\pi(t - \xi)/T_{stim}$, wobei $\xi \neq \tau_1$ und $\xi$ = konst gilt, oder indem eine Phasenverschiebung $\vartheta$ explizit dazu addiert wird: $\varphi_1(t) = 2\pi(t - \tau_1)/T_{stim} + \vartheta$, wobei $\vartheta$ = konst gilt. Die Periode $T_{stim}$ kann auch eine zeitlich langsam variierende Funktion der Zeit sein: $T_{stim} = \chi(t)$. Insbesondere kann die Periode $T_{stim}$ deterministisch oder stochastisch oder gemischt deterministisch-stochastisch langsam in der Zeit variiert werden, wobei die Änderungen der Stimulationsfrequenz $f_{stim}$ im Vergleich zur Zeitskala der zu untersuchenden Oszillation mindestens eine Größenordnung langsamer sind.

Die Phase $\varphi_2(t)$ des zu untersuchenden neuronalen Rhythmus wird aus dem Messsignal 23 und insbesondere mittels Hilbert-Transformation aus dem mittels Bandpassfilterung bzw. "empirical mode decomposition" bestimmten Signal, welches die pathologische oszillatorische Aktivität repräsentiert, ermittelt. Die "empirical mode decomposition" ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454, 903-995 (1998)). Die Kombination "empirical mode decomposition" mit nachfolgender Hilbert-Transformation wird als Hilbert-Huang-Transformation bezeichnet (vgl. N. E. Huang et al.; A confidence limit for the empirical mode decomposition and Hilbert spectral analysis. Proceedings of the Royal Society of London Series A 459, 2317-2345 (2003)). Die Phase $\varphi_2(t)$ des zu untersuchenden neuronalen Rhythmus kann auch mittels wavelet-Analyse bestimmt werden. Neben der instantanen (zeitabhängigen) Phase $\varphi_2(t)$ der zu untersuchenden neuronalen Oszillation erhält man auf diese Weise zusätzlich deren instantane (zeitabhängige) Amplitude.

Um den Grad der Phasensynchronisation zwischen dem Reiz und neuronalem Rhythmus zu bestimmen, betrachten wir die n:m-Phasendifferenz zwischen dem Reiz und dem neuronalen Rhythmus $\psi_{n,m}(t) = n\varphi_1(t) - m\varphi_2(t)$, wobei n und m kleine ganze Zahlen sind. Auf diese Weise kann die Phasensynchronisation zwischen dem Reiz und einem neuronalen Rhythmus in unterschiedlichen Frequenzbändern untersucht werden. D. h., zur Untersuchung des Effekts der Stimulation auf neuronale Rhythmen muss man sich nicht auf den Rhythmus, der sich im selben Frequenzbereich wie die Reizfrequenz (n = m = 1) befindet, beschränken. Die n:m-Phasendifferenz modulo 2n lautet $\Phi_{n,m}(t) = [n\varphi_1(t) - m\varphi_2(t)]_{mod\ 2\pi}$.

Bestimmt u. a. durch die Abtastrate wird zu den Zeiten $t_1, t_2,..., t_N$ die n:m-Phasendifferenz modulo 2n ermittelt. Hiermit erhalten wir die zugehörige Verteilung von $\Phi_{n,m}$, welche $\left\{\Phi_{n,m}(t)\right\}_{j=a}^{b}$ lautet. Die Verteilung kann alle gemessenen Werte von $\Phi_{n,m}$ beinhalten ($a = t_1$, $b = t_N$) oder nur eine Untergruppe ($a > ti$ und/oder $b < t_N$), um z. B. Einschwingvorgänge von der Analyse auszuschließen. Hierzu werden z. B. die ersten ca. 10 Reize aus der Analyse herausgenommen.

Liegt keinerlei n:m-Phasensynchronisation vor, so ist die Verteilung der n:m-Phasendifferenz modulo 2n eine Gleichverteilung (bzw. kommt dieser hinreichend nahe). Im Gegensatz hierzu ist n:m-Phasensynchronisation durch das Auftreten von einem oder mehreren Häufungswerten von $\Phi_{n,m}(t)$ charakterisiert (vgl. P. Tass, M.G. Rosenblum, J. Weule, J. Kurths, A. Pikovsky, J. Volkmann, A. Schnitzler und H.-J. Freund: Detection of n:m Phase Locking from Noisy Data: Application to Magnetoencephalography. Phys. Rev. Lett. 81 (15), 3291-3294 (1998); M. G. Rosenblum, A. S. Pikovsky, C. Schäfer, J. Kurths, P. A. Tass: Phase Synchronization: From Theory to Data Analysis. In: Moss F. (Ed.): Handbook of Biological Physics, Elsevier (2000)).

Das Auftreten einer n:m-Phasensynchronisation kann mittels unterschiedlicher Variablen bestimmt werden. Im Folgenden werden Beispiele angeführt:

(i) Sollte die Verteilung von $\Phi_{n,m}$ einen (einzigen) Häufungswert aufweisen, so kann der zirkuläre Mittelwert dieser Verteilung berechnet werden: $S_{n,m} = \left| \dfrac{1}{b-a+1} \sum_{l=a}^{b} \exp[i\Phi_{n,m}(t_l)] \right|$, wobei $0 \leq S_{n,m} \leq 1$ gilt, und eine Gleichverteilung zu $S_{n,m} = 0$ führt, während eine perfekte Phasensynchronisation durch $S_{n,m} = 1$ gekennzeichnet ist (vgl. M. G. Rosenblum, A. S. Pikovsky, C. Schäfer, J. Kurths, P. A. Tass: Phase Synchronization: From Theory to Data Analysis. In: Moss F. (Ed.): Handbook of Biological Physics, Elsevier (2000)). Der Nachteil dieses Synchronisationsindizes ist, dass er typischerweise keine sinnvollen Ergebnisse liefert, wenn die Verteilung $\left\{\Phi_{n,m}(t)\right\}_{j=a}^{b}$ mehr als einen Häufungswert aufweist. Weist sie z. B. zwei gegenphasige Häufungswerte auf, so ergibt sich ein Wert von $S_{n,m}$ nahe Null.

(ii) Deswegen sollte zudem (oder ausschließlich) ein Synchronisationsindex berechnet werden, der unabhängig von der Anzahl der Häufungswerte verlässliche Ergebnisse liefert. Zu diesem Zweck wird mit dem Kuiper-Test, der zirkulären Variante des Kolmogorov-Smirnov-Tests (vgl. E. Batschelet: Circular Statistics in Biology (Academic Press, London, 1981); N. H. Kuiper: Tests concerning random points in a circle. Proc. K. Ned. Akad. Wet., Ser. A: Math. Sci. 63, 38 (1960); P. A. Tass: Transmission of stimulus-locked responses in two coupled phase oscillators.

Phys. Rev. E 69, 051909-1-24 (2004)), die Wahrscheinlichkeit bestimmt, mit der die Verteilung $\left\{\Phi_{n,m}(t)\right\}_{j=a}^{b}$ eine Gleichverteilung ist. Man erhält dann einen P-Wert, welcher das kleinste Signifikanzniveau ist, mit dem die Nullhypothese (dass die beobachtete Verteilung $\left\{\Phi_{n,m}(t)\right\}_{j=a}^{b}$ eine Gleichverteilung ist) zurückgewiesen werden kann.

Ist $\left\{\Phi_{n,m}(t)\right\}_{j=a}^{b}$ eine Gleichverteilung, ergibt sich P = 1. Hat hingegen $\left\{\Phi_{n,m}(t)\right\}_{j=a}^{b}$ einen einzelnen, deutlich ausgeprägten Häufungswert, so erhält man einen P-Wert nahe 0.

(iii) Eine weitere Möglichkeit ist ein auf der Shannon-Entropie der Verteilung $\left\{\Phi_{n,m}(t)\right\}_{j=a}^{b}$ basierender n:m-Synchronisationsindex $\rho_{n,m}$. Mit einer Abschätzung $p_k$ der Verteilung $\left\{\Phi_{n,m}(t)\right\}_{j=a}^{b}$ ergibt sich $\rho_{n,m} = \dfrac{S_{max} - S}{S_{max}}$,

wobei $S = -\sum_{k=1}^{L} p_k \ln p_k$ gilt. Die maximale Entropie lau-tet $S_{max}$ = lnL, wobei L die Anzahl der bins ist. $p_k$ ist die relative Häufigkeit, mit der $\Phi_{n,m}$ im k-ten bin gefunden wird (vgl. P. Landa: Nonlinear Oscillations and Waves in Dyanmical Systems. Kluwer Academic Publishers, Dordrecht-Boston-London, 1996). Aufgrund der Normalisierung gilt $0 \leq \rho_{n,m} \leq 1$. Bei einer Gleichverteilung, also dem völligen Fehlen einer n:m-Phasensynchronisation, ergibt sich $\rho_{n,m}$ = 0, während eine perfekte n:m-Phasensynchronisation zu einer Dirac-artigen Verteilung führt (d. h., alle Werte von $\Phi_{n,m}$ liegen im selben bin), so dass $\rho_{n,m}$ = 1 gilt. Im Vergleich zu dem unter (ii) angeführten, auf dem Kuiper-Test basierenden n:m-Synchronisations-index hat der auf der Shannon-Entropie basierende n:m-Synchronisationsindex $\rho_{n,m}$ den Nachteil, dass sein Wert bei stärker ausgeprägten Häufungswerten der Verteilung von der genauen Lage des Häufungswertes abhängt, so dass sich bei einer zyklischen Verschiebung des Häufungswertes im Intervall [0,2n] artefizielle Oszillationen ergeben (vgl. P. A. Tass: Transmission of stimulus-locked responses in two coupled phase oscillators. Phys. Rev. E 69, 051909-1-24 (2004)).

Neben den stimulationsbedingten Effekten auf die Phase $\varphi_2(t)$ des neuronalen Rhythmus und damit die n:m-Phasendifferenz $\Phi_{n,m}$ werden auch stimulationsbedingte Effekte auf die Amplitude A(t) des neuronalen Rhythmus untersucht. n und m ergeben sich aus dem Verhältnis von Stimulationsfrequenz $f_{stim}$ zu dem dominanten spektralen Frequenzpeak. Die Analyse kann aber auch standardmäßig für einige wenige Paare von n und m (jeweils klein und ganzzahlig, z. B. n, m = 1, 2, 3) durchgeführt werden, z. B.: (n,m) = (1,1) = (1,2) = (2,1) = (2,3) = (3,2) =.... Es geht nicht nur darum, ob die Stimulation den neuronalen Rhythmus in einem n:m-Verhältnis in Takt bringt, sondern ob die Stimulation auch das Ausmaß der dem neuronalen Rhythmus zugrunde liegenden Synchronisation der Neuronen verändert. Ein Anstieg der Synchronisation innerhalb der stimulierten Neuronenpopulation führt zu einem Anwachsen der Amplitude A(t). Umgekehrt führt eine Abnahme der Synchronisation innerhalb der dem neuronalen Rhythmus zugrunde liegenden Neuronenpopulation zu einer Abnahme der Amplitude A(t). Für die Auswertung der Amplitudeneffekte sind somit Messungen in einem Vergleichsintervall (z. B. vor Verabreichung der Stimulation) durchzuführen. Alternativ können Amplitudeneffekte auch einfach durch eine Analyse der spektralen Leistungsdichte - eines vordefinierten, für die Erkrankung typischen und dem Fachmann bekannten Frequenzbandes oder einer bzw. mehrerer aus den Daten extrahierten empirical modes oder mittels wavelet-Analyse bestimmt werden.

Zur Bestimmung der Signifikanz der durch die periodische Reizabfolge induzierten Phaseneffekte bzw. Amplitudeneffekte kann eine prä-Stimulus-Baseline in einem Zeitfenster $F_{pre}$ mit einer Länge, die dem Zehn- bis Tausendfachen der mittleren Periode der zu untersuchenden Oszillation entspricht, bestimmt werden:

Zur Baseline-Bestimmung der Amplitude A(t) des neuronalen Rhythmus wird in dem Zeitfenster $F_{pre}$ z. B. die 99te Perzentile dieser Verteilung oder einfach das Maximum als Baselinewert für A(t) genommen. Falls die Signale wegen Artefakten nicht von bester Qualität sein sollten, weil z. B. bei EEG-Signalen keine Artefakt-Elimination vorgeschaltet wurde, kann man auch eine niedrigere Perzentile als Baseline wählen (falls die Artefakte große Amplitudenwerte haben sollten). Ein derartiges Vorgehen ist dem Fachmann vertraut.

Eine Baseline der n:m-Phasensynchronisation kann z. B. dadurch bestimmt werden, indem in dem Zeitfenster $F_{pre}$ sozusagen eine Sham-Stimulation durchgeführt wird. D. h., in dem Zeitfenster $F_{pre}$, in dem nicht stimuliert wird, wird eine n:m-Phasensynchronisation mit einer virtuellen periodischen Reizabfolge durchgeführt. Eine andere Möglichkeit zur Bestimmung wäre z. B., die echten unter Stimulation abgeleiteten Signale durch Surrogat-Daten zu ersetzen und hierfür dann die n:m-Phasensynchronisation zu bestimmen. Derartige Prozeduren sind dem Fachmann bekannt.

Nachdem mit Hilfe des Entrainment-Tests Aktivierungsgruppen, d. h. Bereiche auf der Körperoberfläche des Patienten, ermittelt wurden, in welchen die Phasensynchronisation zwischen der periodischen Applikation der Reize und der neuronalen Aktivität der stimulierten Neuronen ausreichend hoch ist bzw. ein lokales Maximum aufweist, wird ein Paar-Test vorgenommen, bei dem phasenrücksetzende Reize in zwei verschiedenen Bereichen auf der Körperoberfläche des Patienten appliziert werden, um wirksame Paare für die CR-Stimulation zu selektieren. Der Paar-Test wird direkt nach dem Entrainment-Test durchgeführt, ohne dass zuvor noch optimale Einzelreize mittels einer "Phase resetting"-Analyse ermittelt werden.

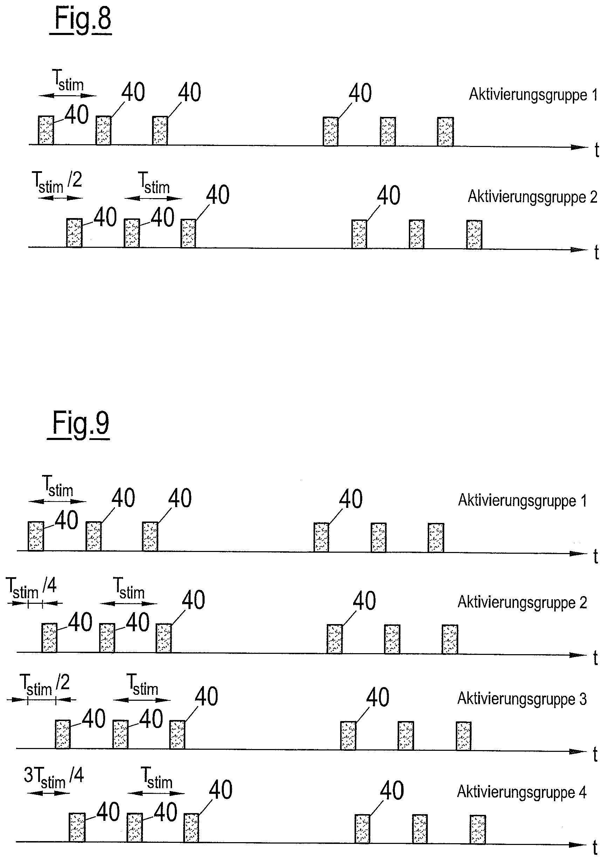

Fig. 8 zeigt schematisch einen Paar-Test. Hier werden zwei zuvor mit Hilfe des Entrainment-Tests ausgewählte Aktivierungsgruppen, d. h. Bereiche auf der Körperoberfläche des Patienten herangezogen, um mechanisch taktile und/oder thermische Reize 40 in dem jeweiligen Bereich auf der Körperoberfläche zu applizieren. Aufgrund der somatotopen Zuordnung stimulieren die in den ausgewählten Bereichen applizierten Reize 40 eine jeweilige Subpopulationen der Neuronenpopulation mit der krankhaft synchronen und oszillatorischen Aktivität. Die Reize 40 sind dazu ausgelegt, die Phase der krankhaft synchronen und oszillatorischen neuronalen Aktivität der jeweils stimulierten Subpopulation zurückzusetzen. Sofern als Ergebnis des Entrainment-Tests mehr als zwei Aktivierungsgruppen ausgewählt wurden, wird der Paar-Test bevorzugt mit jeweils benachbarten Aktivierungsgruppen durchgeführt.

Die Stimulationselemente 35 in jeder der beiden Aktivierungsgruppen 1 und 2 erzeugen die phasenrücksetzenden Reize 40 in einer Sequenz periodisch mit der Periode $T_{stim}$. In dem vorliegenden Fall umfasst jede Sequenz drei Reize 40, die Sequenzen können allerdings auch weitere Reize 40 enthalten. Nach jeder Sequenz wird eine vorgegebene Pause eingehalten und die Sequenz wird danach wiederholt. Die Pause, auf die auch verzichtet werden kann, kann insbesondere ein ganzzahliges Vielfaches der Periode $T_{stim}$ betragen. Ferner beträgt die zeitliche Verzögerung zwischen den Sequenzen unterschiedlicher Aktivierungsgruppen $T_{stim}/2$, d. h., die Stimulation der Aktivierungsgruppe 2 ist um $T_{stim}/2$ zeitversetzt zur Stimulation der Aktivierungsgruppe 1, so dass die Stimulation der jeweiligen Subpopulationen ebenfalls zeitversetzt erfolgt. Aufgrund dieses Zeitversatzes werden die Phasen der stimulierten Subpopulationen zu unterschiedlichen Zeitpunkten zurückgesetzt. Bis auf den Zeitversatz können die von den Aktivierungsgruppen 1 und 2 durchgeführten Stimulationen z. B. identisch sein.

Ob über ein jeweiliges Paar von Aktivierungsgruppen effektiv stimuliert werden kann, bemisst sich an der von der Messeinheit 12 gemessenen Amplitude A(t) der pathologischen Oszillation. Eine paarweise Stimulation gilt dann als erfolgreich, wenn kein Amplitudenanstieg A(t) der pathologischen Oszillation (also keine Verstärkung der Synchronisation der das Signal generierenden pathologisch synchronisierten Neuronenpopulation) oder sogar eine leichte Abnahme (entspricht einer schwachen Desynchronisation) auftritt. D. h., die Amplitude A(t) des neuronalen Rhythmus darf im Vergleich zu einem Baseline-Zeitfenster $F_{pre}$ nicht anwachsen. Vielmehr sollte die Amplitude A(t) im Vergleich zu dem Baseline-Zeitfenster $F_{pre}$ abfallen oder zumindest unverändert bleiben. Man kann den unter Paar-Stimulation erzielten Wert der Amplitude A(t) z. B. einer Perzentile der Verteilung der Amplitude A(t) im Baseline-Zeitfenster $F_{pre}$ zuordnen. Oder man kann entsprechende Verhältnisse berechnen.

Ein Paar-Test wird entweder wird für alle möglichen Paare, die aus den ausgewählten Aktivierungsgruppen gebildet werden können, durchgeführt oder, da schneller, z. B. entlang einer Vorzugsachse, z. B. entlang eines am Bauch getragenen Gürtels oder von den Fingern in Richtung Schultern, vorgehend, von der ersten Aktivierungsgruppe zu der nächsten nächst geeigneten Aktivierungsgruppe - und von dieser dann zur wieder nächsten usw. Dies spart Zeit. Man kann z. B. auch aus der Matrix aller Paar-Vergleiche die besten Dreier-, Vierer-, Fünfer-, Sechser- (usw., z. B. bis zu Zehner-) Gruppen bestehend aus 3, 4, 5, 6,..., 10 Aktivierungsgruppen, d. h. Bereichen auf der Körperoberfläche des Patienten, extrahieren. Ungeeignete Paare von Aktivierungsgruppen, die z. B. die pathologisehe neuronale Synchronisation verstärken, werden ersetzt durch besser geeignete Paare.

Die auf diese Weise bestimmten Gruppen werden im Gruppen-Test bzgl. ihrer Effektivität untersucht. Ein beispielhafter Gruppen-Test einer aus vier Aktivierungsgruppen bestehenden Gruppe ist beispielhaft in Fig. 9 gezeigt. Jede aus den jeweiligen Stimulationselementen 35 bestehende Aktivierungsgruppe appliziert genauso wie beim Paar-Test eine Sequenz von phasenrücksetzenden Reizen 40 in dem jeweiligen Bereich auf der Körperoberfläche des Patienten. Die phasenrücksetzenden Reize 40 werden innerhalb einer Sequenz periodisch mit der Periode $T_{stim}$ appliziert. In dem vorliegenden Beispiel umfasst jede Sequenz drei Reize 40, die Sequenzen können allerdings auch mehr Reize 40 enthalten. Nach jeder Sequenz wird eine bestimmte Pause eingehalten und die Sequenz danach wiederholt. Alternativ kann auf die Pause auch verzichtet werden. Ferner beträgt die zeitliche Verzögerung zwischen den Sequenzen benachbarter Aktivierungsgruppen $T_{stim}/4$, da vier Aktivierungsgruppen in dem Gruppen-Test ausgetestet werden. Für den allgemeinen Fall von N Aktivierungsgruppen würde die zeitliche Verzögerung benachbarter Kanäle $T_{stim}$/N betragen. Weiterhin ist es möglich, dass die Reihenfolge, in welcher die Aktivierungsgruppen die Reize 40 erzeugen, variiert wird. Ferner können die von den N Aktivierungsgruppen erzeugten Reize 40 z. B. identisch sein.

Die Steuer- und Analyseeinheit 10 prüft, ob bei der Applikation der Reize 40 über die ausgewählte Gruppe von Aktivierungsgruppen die krankhaft synchrone und oszillatorische neuronale Aktivität der stimulierten Neuronen unterdrückt und insbesondere desynchronisiert wird. Die Effektivität der CR-Stimulation über die wie oben beschrieben ermittelten Gruppen wird mit dem wie beim Paar-Test beschriebenen Amplitudenkriterium und/oder mittels klinischer Bestimmung, z. B. mittels klinischer Scores oder einfacher klinischer Beobachtung durch einen erfahrenen Arzt oder sonstigen Therapeuten, bestimmt. Sollte keine der ausgewählten Gruppen effektiv sein, müssen neue Gruppen anhand der Paar-Matrix ausgesucht werden, bzw. es muss die volle Paar-Matrix bestimmt werden.

Gemäß einer Ausgestaltung wird auf den Paar-Test verzichtet und nach Abschluss des Entrainment-Tests wird sogleich ein Gruppen-Test durchgeführt. Alle, z. B. vier, im Zuge des Entrainment-Test ausgewählten Aktivierungsgruppen werden verwandt, um eine CR-Stimulation zu applizieren. Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 12, z. B. EEG-Elektroden oder Tiefelektroden (als LFP-Signal), gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Reize 40 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sog. minimalen klinisch wahrnehmbaren Verbesserung) sind dem Fachmann bekannt.

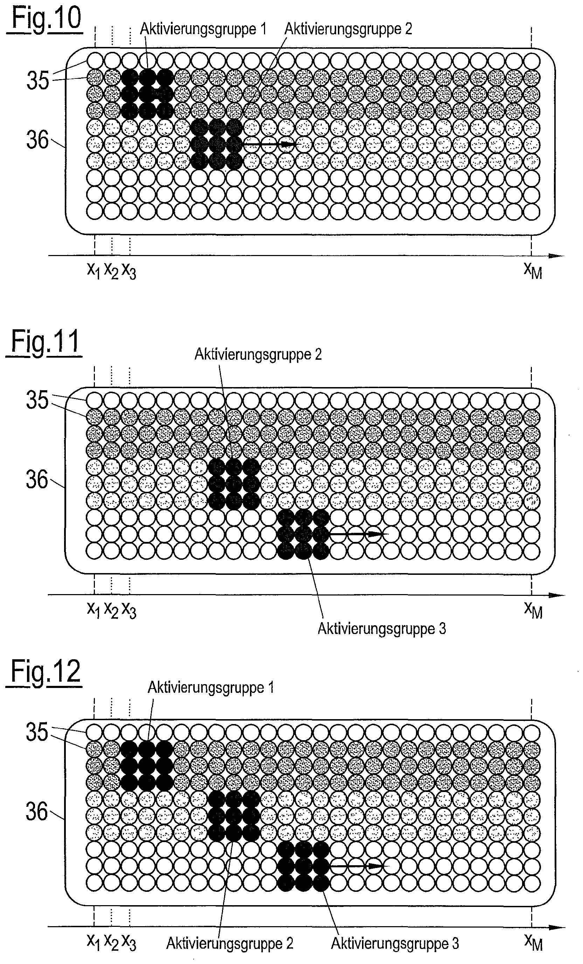

In einer anderen Ausgestaltung wird von einer geeigneten (Ausgangs-) Aktivierungsgruppe, in Fig. 10 ist dies beispielhaft die Aktivierungsgruppe 1, ausgehend eine zweite Aktivierungsgruppe, in Fig. 10 die dies beispielhaft die Aktivierungsgruppe 2, eingeführt und langsam von der Ausgangs-Aktivierungsgruppe weg (wie in Fig. 10) bzw. zu dieser hin (nicht gezeichnet) bewegt. Währenddessen wird die in Fig. 8 gezeigte Stimulation durchgeführt, mit welcher die von den Aktivierungsgruppen 1 und 2 zu einem jeweiligen Zeitpunkt bedeckten Bereiche auf der Körperoberfläche des Patienten stimuliert werden. Der Pfad der Bewegung kann dabei vorgegeben oder wie oben erläutert vom Anwender eingestellt werden. Der Ausgangspunkt der Aktivierungsgruppe 2 kann nahe bei der Aktivierungsgruppe 1 liegen oder mit dem Ausgangspunkt der Aktivierungsgruppe 1 koinzidieren. Eine geeignete Entfernung beider Aktivierungsgruppen erkennt man durch eine guten Stimulationseffekt (elektrophysiolgisch eine gute Desynchronisation bzw. klinisch eine gute Symptomreduktion). Sobald dies erreicht ist, sollte der Abstand nicht weiter vergrößert werden, um insgesamt hinreichend viele Aktivierungsgruppen für die nachfolgende therapeutische Stimulation nutzen zu können.

Iterativ kann man bei dieser Methode nun prinzipiell zwei Wege beschreiten. Eine erste Möglichkeit ist die paarweise Testung weiterer geeigneter Paare von Aktivierungsgruppen, d. h., die gerade ermittelte Aktivierungsgruppe 2 wird zur neuen Ausgangs-Aktivierungsgruppe, wie dies in Fig. 11 gezeigt ist, und es wird eine weitere Aktivierungsgruppe, nämlich die Aktivierungsgruppe 3 hinzugenommen. Die Aktivierungsgruppe 3 wird langsam von der Ausgangs-Aktivierungsgruppe 2 weg oder zu dieser hin bewegt, wobei mit den Aktivierungsgruppen 2 und 3 die in Fig. 8 gezeigte Stimulation durchgeführt wird (mit N = 2), bis anhand der von der Messeinheit 12 aufgenommenen Messsignale 23 festgestellt wird, dass ein guter Stimulationseffekt erzielt wird, z. B. eine mit Hilfe der Messeinheit 12 beobachtete gute Desynchronisation vorliegt. Der Ausgangspunkt der Aktivierungsgruppe 3 kann nahe bei der Aktivierungsgruppe 2 liegen oder mit dem Ausgangspunkt der Aktivierungsgruppe 3 koinzidieren. Sind nacheinander schließlich alle geeigneten Paare von Aktivierungsgruppen ermittelt worden, wird mit diesen allen sowie Untergruppen von Aktivierungsgruppen eine CR-Stimulation durchgeführt, wie dies in Fig. 9 gezeigt ist. Die Gruppe bzw. Subgruppe, welche die besten Ergebnisse liefert, wird für die nachfolgende therapeutische Stimulation ausgewählt.

Für die praktische Anwendung ist es schneller, wenn man iterativ die Anzahl der Aktivierungsgruppen erweitert. Im Beispiel von 10 sind die beiden als geeignet ermittelten Aktivierungsgruppen 1 und 2 gesetzt. Es wird eine Aktivierungsgruppe 3 hinzugenommen, und deren Abstand entlang eines vorgegebenen oder vom Anwender gewählten Pfads variiert, wie dies beispielhaft in Fig. 12 gezeigt ist. Die Aktivierungsgruppe 3 wird langsam von der Ausgangs-Aktivierungsgruppe 2 weg oder zu dieser hin bewegt, wobei währenddessen die in Fig. 9 gezeigte Stimulation (mit N = 3, da mit drei Aktivierungsgruppen stimuliert wird) durchgeführt wird, bis anhand der von der Messeinheit 12 aufgenommenen Messsignale 23 festgestellt wird, dass ein guter Stimulationseffekt erzielt wird, z. B. eine mit Hilfe der Messeinheit 12 beobachtete gute Desynchronisation vorliegt. Der Ausgangspunkt der Aktivierungsgruppe 3 kann nahe bei der Aktivierungsgruppe 2 liegen oder mit den Ausgangspunkt der Aktivierungsgruppe 3 koinzidieren. Im Gegensatz zur in Fig. 11 gezeigten paarweisen Testung werden bei der in Fig. 12 gezeigten Ausführungsform alle zuvor ermittelten Aktivierungsgruppen beibehalten. Auf diese Weise wird schließlich das gesamte von der Manschette 36 bedeckte Körperteil mit Aktivierungsgruppen abgedeckt. Die hierdurch erhaltene Konfiguration wird für die nachfolgende therapeutische Stimulation verwandt.

Zur Überprüfung, ob die in den Ausgestaltungen nach den Fig. 8 bis 12 applizierten Reize 40 die Phase der neuronalen Aktivität zurücksetzen, sind dem Fachmann verschiedene Analyseverfahren vertraut. Die Analyse der Phasenrücksetzung der synchronen neuronalen Aktivität erfolgt typischerweise mittels eines Ensembles von identischen Reizen. Eine Möglichkeit, die dem Fachmann zur Analyse der Phasenrücksetzung geläufig ist, besteht aus einer "phase resetting"-Analyse, wie sie beispielsweise in dem Artikel "Stochastic phase resetting of two coupled phase oscillators stimulated at different times" von P. A. Tass (erschienen in Physical Review E 67, 2003, Seiten 051902-1 bis 051902-15) beschrieben ist. Dazu wird der "phase resetting"-Index ermittelt (vgl. Gleichung 8, "stimulus-locking index" für v = 1). Die hierbei zur Berechnung der Phasenrücksetzung verwandte Phase wird z. B. mittels der Hilbert-Transformation aus dem mittels Bandpassfilterung bzw. "empirical mode decomposition" bestimmten Signal, welches die pathologische oszillatorische Aktivität repräsentiert, ermittelt (letztere ermöglicht im Vergleich zur Bandpassfilterung eine parameterunabhängige Bestimmung physiologisch relevanter Moden in verschiedenen Frequenzbereichen, vgl. "The empirical mode decomposition and the Hilbert spectrum for nonlinear and non-stationary time series analysis" von N. E. Huang et al. (erschienen in Proceedings of the Royal Society of London Series A, 1998, Band 454, Seiten 903 bis 995); die Kombination "empirical mode decomposition" mit nachfolgender Hilbert-Transformation wird als Hilbert-Huang-Transformation bezeichnet, vgl. "A confidence limit for the empirical mode decomposition and Hilbert spectral analysis" von N. E. Huang et al. (erschienen in Proceedings of the Royal Society of London Series A, 2003, Band 459, Seiten 2317 bis 2345). Eine Phasenrücksetzung ist erreicht, wenn der "phase resetting"-Index die 99. Perzentile der prä-Stimulus-Verteilung des "phase resetting"-Index überschreitet (vgl. Fig. 4 in "Stochastic phase resetting of two coupled phase oscillators stimulated at different times" von P. A. Tass). Falls medizinisch eine stärkere phasenrücksetzende Wirkung wünschenswert ist, können auch höhere Schwellen, z. B. das Doppelte oder das Dreifache der 99. Perzentile der prä-Stimulus-Verteilung des "phase resetting"-Index gewählt werden.

Alternativ zu dieser Datenanalyse können auch einfachere Datenanalyse-Verfahren angewandt werden, die die Detektion der Phasenrücksetzung mit in der Praxis hinreichender Genauigkeit zu approximieren vermögen. Z. B. kann einfach über das Ensemble an Reizantworten gemittelt werden. Von einer Phasenrücksetzung ist dann approximativ auszugehen, wenn der maximale Betrag der Reizantwort die 99. Perzentile der prä-Stimulus-Verteilung der gemittelten Antwort (oder deren Doppeltes bzw. Dreifaches) überschreitet (vgl. Fig. 6 in "Stochastic phase resetting of two coupled phase oscillators stimulated at different times" von P. A. Tass, Phys. Rev. E 67, 051902-1 bis 051902-15 (2003)).

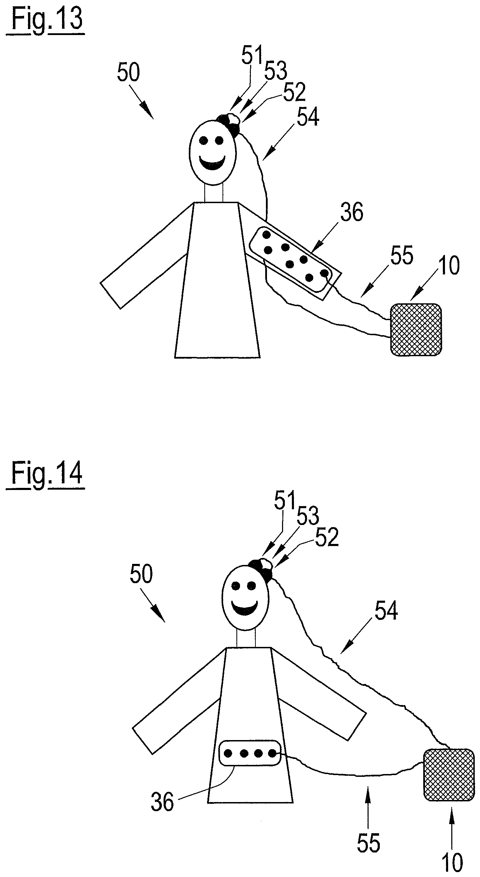

Fig. 13 zeigt schematisch eine Vorrichtung 50 zur EEG-basierten Kalibrierung einer mechanisch taktilen und/oder thermischen CR-Stimulation. Nicht-invasiv fixierte EEG-Elektroden 51, 52, die über ein Kabel 53 verbunden sind, dienen als Messeinheit und messen die EEG-Reizantworten, welche über ein Kabel 54 an die zentrale Steuer- und Analyseeinheit 10 weitergeleitet werden. Eine Manschette 36 mit einem Array aus Stimulationselementen 35 ist am Unterarm des Patienten mit Klettverschlüssen fixiert. Die mittels der oben beschriebenen Kalibrierung ausgewählten Aktivierungsgruppen sind durch Punkte gekennzeichnet. Die Manschette 36 ist über ein Kabel 55 oder telemetrisch mit der die Batterie bzw. den Akku beherbergenden Steuer- und Analyseeinheit 10 verbunden. Im Falle der telemetrischen Verbindung beherbergt die Manschette 36 auch eine Batterie bzw. einen Akku.

Fig. 14 zeigt die Vorrichtung 50 aus Fig. 13, wobei die Manschette 36 hier gürtelförmig am Bauch des Patienten befestigt ist.

**Patentansprüche**

1. Vorrichtung (1) zur Stimulation von Neuronen, umfassend

- eine nicht-invasive Stimulationseinheit (11) zur Applikation von mechanisch taktilen und/oder thermischen Reizen (22) auf die Körperoberfläche eines Patienten, wobei die Reize (22) Neuronen mit einer krankhaft synchronen und oszillatorischen neuronalen Aktivität stimulieren,
- eine Messeinheit (12) zur Aufnahme von Messsignalen (23), die eine neuronale Aktivität der stimulierten Neuronen wiedergeben, und
- eine Steuer- und Analyseeinheit (10) zur Steuerung der Stimulationseinheit (11) und zur Analyse der Messsignale (23), wobei die Steuer- und Analyseeinheit (10) derart ausgestaltet ist, dass sie
- die Stimulationseinheit (11) derart ansteuert, dass diese zumindest einen Teil der Körperoberfläche des Patienten entlang eines Pfads abrastert und dabei periodisch Reize appliziert,
- anhand der in Reaktion auf die periodische Applikation der Reize aufgenommenen Messsignale mindestens zwei Bereiche auf der Körperoberfläche des Patienten entlang des Pfads auswählt, in welchen die Phasensynchronisation zwischen der periodischen Applikation der Reize und der neuronalen Aktivität der stimulierten Neuronen jeweils ein lokales Maximum aufweist, und
- die Stimulationseinheit (11) derart ansteuert, dass diese in den mindestens zwei ausgewählten Bereichen zeitversetzt Reize (40) appliziert, die eine Phasenrücksetzung der neuronalen Aktivität der stimulierten Neuronen bewirken.

2. Vorrichtung (1) nach Anspruch 1, wobei die Steuer- und Analyseeinheit (10) anhand der in Reaktion auf die zeitversetzt applizierten Reize (40) aufgenommenen Messsignale (23) prüft, ob die zeitversetzt applizierten Reize (40) die krankhaft synchrone und oszillatorische Aktivität der stimulierten Neuronen reduzieren.

3. Vorrichtung (1) nach Anspruch 1 oder 2, wobei die von der Steuer- und Analyseeinheit (10) in Reaktion auf die periodische Applikation der Reize ausgewählten mindestens zwei Bereiche einen ersten Bereich und einen zweiten Bereich umfassen und wobei die Steuer- und Analyseeinheit (10) den zweiten Bereich auf der Körperoberfläche des Patienten verschiebt, falls die Steuer- und Analyseeinheit (10) anhand der in Reaktion auf die zeitversetzt applizierten Reize (40) aufgenommenen Messsignale (23) feststellt, dass die zeitversetzt applizierten Reize (40) die krankhaft synchrone und oszillatorische Aktivität der stimulierten Neuronen nicht reduzieren.

4. Vorrichtung (1) nach Anspruch 3, wobei die Steuer- und Analyseeinheit (10) den zweiten Bereich auf der Körperoberfläche des Patienten solange verschiebt, bis die Steuer- und Analyseeinheit (10) anhand der in Reaktion auf die zeitversetzt applizierten Reize (40) aufgenommenen Messsignale (23) feststellt, dass die zeitversetzt applizierten Reize (40) die krankhaft synchrone und oszillatorische Aktivität der stimulierten Neuronen reduzieren.

5. Vorrichtung (1) nach Anspruch 3 oder 4, wobei die Steuer- und Analyseeinheit (10) nach der Applikation der zeitversetzten Reize (40) in dem ersten und dem zweiten Bereich einen dritten Bereich auf der Körperoberfläche des Patienten auswählt und die Stimulationseinheit (11) derart ansteuert, dass diese in dem zweiten Bereich und dem dritten Bereich, jedoch nicht in dem ersten Bereich zeitversetzt Reize (40) appliziert, die eine Phasenrücksetzung der neuronalen Aktivität der stimulierten Neuronen bewirken.

6. Vorrichtung (1) nach Anspruch 5, wobei die Steuer- und Analyseeinheit (10) den dritten Bereich auf der Körperoberfläche des Patienten verschiebt, falls die Steuer- und Analyseeinheit (10) anhand der in Reaktion auf die zeitversetzt in dem zweiten und dem dritten Bereich applizierten Reize (40) aufgenommenen Messsignale (23) feststellt, dass die zeitversetzt applizierten Reize (40) die krankhaft synchrone und oszillatorische Aktivität der stimulierten Neuronen nicht reduzieren, und/oder wobei die Steuer- und Analyseeinheit (10) nach der Applikation der zeitversetzten Reize (40) in dem zweiten und dem dritten Bereich einen oder mehrere weitere Bereiche auf der Körperoberfläche des Patienten auswählt und die Stimulationseinheit (11) derart ansteuert, dass diese in zwei der ausgewählten Bereiche, jedoch nicht in den übrigen Bereichen zeitversetzt Reize (40) appliziert, die eine Phasenrücksetzung der neuronalen Aktivität der stimulierten Neuronen bewirken.

7. Vorrichtung (1) nach Anspruch 3 oder 4, wobei die Steuer- und Analyseeinheit (10) nach der Applikation der zeitversetzten Reize (40) in dem ersten und dem zweiten Bereich einen dritten Bereich auf der Körperoberfläche des Patienten auswählt und die Stimulationseinheit (11) derart ansteuert, dass diese in dem ersten Bereich, dem zweiten Bereich und dem dritten Bereich zeitversetzt Reize (40) appliziert, die eine Phasenrücksetzung der neuronalen Aktivität der stimulierten Neuronen bewirken.

8. Vorrichtung (1) nach Anspruch 7, wobei die Steuer- und Analyseeinheit (10) den dritten Bereich auf der Körperoberfläche des Patienten verschiebt, falls die Steuer- und Analyseeinheit (10) anhand der in Reaktion auf die zeitversetzt in dem ersten, dem zweiten und dem dritten Bereich applizierten Reize (40) aufgenommenen Messsignale (23) feststellt, dass die zeitversetzt applizierten Reize (40) die krankhaft synchrone und oszillatorische Aktivität der stimulierten Neuronen nicht reduzieren.

9. Vorrichtung (1) nach Anspruch 7 oder 8, wobei die Steuer- und Analyseeinheit (10) nach der Applikation der zeitversetzten Reize (40) in dem ersten, dem zweiten und dem dritten Bereich einen oder mehrere weitere Bereiche auf der Körperoberfläche des Patienten auswählt und die Stimulationseinheit (11) derart ansteuert, dass diese in dem ersten Bereich, dem zweiten Bereich, dem dritten Bereich und dem oder den weiteren ausgewählten Bereichen zeitversetzt Reize (40) appliziert, die eine Phasenrücksetzung der neuronalen Aktivität der stimulierten Neuronen bewirken.

10. Vorrichtung (1) nach einem der vorhergehenden Ansprüche, wobei die Stimulationseinheit (11) eine Mehrzahl von in einem Array angeordneten Stimulationselementen (35) aufweist, welche die mechanisch taktilen und/oder thermischen Reize (22) erzeugen.

**11.** Vorrichtung (1) nach Anspruch 10, wobei eine Aktivierungsgruppe diejenigen Stimulationselemente (35) umfasst, die sich zumindest teilweise innerhalb einer bestimmten, auf dem Array darstellbaren Form befinden, wobei bei einer Bewegung der Aktivierungsgruppe die Form sich über das Array bewegt und die von der Aktivierungsgruppe umfassten Stimulationselemente (35) sich entsprechend ändern.

**12.** Vorrichtung (1) nach Anspruch 11, wobei bei der Abrasterung zumindest eines Teils der Körperoberfläche des Patienten durch die Stimulationseinheit (11) die Aktivierungsgruppe entlang des Pfads über das Array wandert und die von der Aktivierungsgruppe umfassten Stimulationselemente (35) die periodischen Reize erzeugen.

**13.** Vorrichtung (1) nach einem der Ansprüche 10 bis 12, wobei die in dem Array angeordneten Stimulationselemente (35) an einer Manschette (36) befestigt sind, die derart ausgestaltet ist, dass sie am Körper des Patienten fixierbar ist.

**14.** Vorrichtung (1) nach einem der Ansprüche 11 bis 13, umfassend eine Eingabeeinheit (37), welche derart ausgestaltet ist, dass ein Benutzer in die Eingabeeinheit (37) die Form der Aktivierungsgruppe und/oder den Pfad, entlang dem die Aktivierungsgruppe wandert, eingeben kann.

**15.** Software zum Ausführen in einem Datenverarbeitungssystem in der Vorrichtung von Anspruch 1, wobei die Software

- Steuersignale zum Ansteuern einer nicht-invasiven Stimulationseinheit (11) erzeugt, wobei die Stimulationseinheit (11) mechanisch taktile und/oder thermische Reize (22) auf die Körperoberfläche eines Patienten appliziert, wobei die Reize (22) Neuronen mit einer krankhaft synchronen und oszillatorischen neuronalen Aktivität stimulieren,
- Messsignale (23) aufgenommen werden, die eine neuronale Aktivität der stimulierten Neuronen wiedergeben,
- die Stimulationseinheit (11) zumindest einen Teil der Körperoberfläche des Patienten entlang eines Pfads abrastert und dabei periodisch Reize appliziert,
- anhand der in Reaktion auf die periodische Applikation der Reize aufgenommenen Messsignale mindestens zwei Bereiche auf der Körperoberfläche des Patienten entlang des Pfads ausgewählt werden, in welchen die Phasensynchronisation zwischen der periodischen Applikation der Reize und der neuronalen Aktivität der stimulierten Neuronen jeweils ein lokales Maximum aufweist, und
- die Stimulationseinheit (11) in den mindestens zwei ausgewählten Bereichen zeitversetzt Reize (40) appliziert, die eine Phasenrücksetzung der neuronalen Aktivität der stimulierten Neuronen bewirken.

**Claims**

**1.** An apparatus (1) for stimulating neurons, comprising

- a non-invasive stimulation unit (11) for the application of mechanically tactile and/or thermal stimuli (22) to the surface of the body of a patient, wherein the stimuli (22) stimulate neurons having a pathologically synchronous and oscillatory neuronal activity;
- a measuring unit (12) for recording measured signals (23) which reproduce a neuronal activity of the stimulated neurons; and
- a control and analysis unit (10) for controlling the stimulation unit (11) and for analyzing the measured signals (23), wherein the control and analysis unit (10) is configured such that it
- controls the stimulation unit (11) such that it scans at least a part of the surface of the body of the patient along a path and in so doing periodically applies stimuli;
- selects at least two regions on the surface of the body of the patient along the path with reference to the measured signals recorded in response to the periodic application of the stimuli, with the phase synchronization between the periodic application of the stimuli and the neuronal activity of the stimulated neurons respectively having a local maximum in said regions; and
- controls the stimulation unit (11) such that it applies stimuli (40) in a time-offset manner in the at least two selected regions, the stimuli effecting a phase reset of the neuronal activity of the stimulated neurons.

**2.** An apparatus (1) in accordance with claim 1, wherein the control and analysis unit (10) checks with reference to the measured signals (23) recorded in response to the stimuli (40) applied in a time-offset manner whether the stimuli (40) applied in a time-offset manner reduce the pathologically synchronous and oscillatory activity of the stimulated neurons.

3. An apparatus (1) in accordance with claim 1 or claim 2, wherein the at least two regions selected by the control and analysis unit (10) in response to the periodic application of the stimuli comprise a first region and a second region; and wherein the control and analysis unit (10) displaces the second region on the surface of the body of the patient if the control and analysis unit (10) determines with reference to the measured signals (23) recorded in response to the stimuli (40) applied in a time-offset manner that the stimuli (40) applied in a time-offset manner do not reduce the pathologically synchronous and oscillatory activity of the stimulated neurons.

4. An apparatus (1) in accordance with claim 3, wherein the control and analysis unit (10) displaces the second region on the surface of the body of the patient for so long until the control and analysis unit (10) determines with reference to the measured signals (23) recorded in response to the stimuli (40) applied in a time-offset manner that the stimuli (40) applied in a time-offset manner reduce the pathologically synchronous and oscillatory activity of the stimulated neurons.

5. An apparatus (1) in accordance with claim 3 or claim 4, wherein the control and analysis unit (10) selects a third region on the surface of the body of the patient after the application of the time-offset stimuli (40) in the first and second regions and controls the stimulation unit (11) such that it applies stimuli (40) in a time-offset manner in the second region and in the third region, but not in the first region, said stimuli effecting a phase reset of the neuronal activity of the stimulated neurons.

6. An apparatus (1) in accordance with claim 5, wherein the control and analysis unit (10) displaces the third region on the surface of the body of the patient if the control and analysis unit (10) determines with reference to the measured signals (23) recorded in response to the stimuli (40) applied in a time-offset manner in the second and third regions that the stimuli (40) applied in a time-offset manner do not reduce the pathologically synchronous and oscillatory activity of the stimulated neurons, and/or wherein the control and analysis unit (10) selects one or more further regions on the surface of the body of the patient after the application of the time-offset stimuli (40) in the second and third regions and controls the stimulation unit (11) such that it applies stimuli (40) in a time-offset manner in two of the selected regions, but not in the remaining regions, said stimuli effecting a phase reset of the neuronal activity of the stimulated neurons.

7. An apparatus (1) in accordance with claim 3 or claim 4, wherein the control and analysis unit (10) selects a third region on the surface of the body of the patient after the application of the time-offset stimuli (40) in the first and second regions and controls the stimulation unit (11) such that it applies stimuli (40) in a time-offset manner in the first region, in the second region and in the third region, said stimuli effecting a phase reset of the neuronal activity of the stimulated neurons.

8. An apparatus (1) in accordance with claim 7, wherein the control and analysis unit (10) displaces the third region on the surface of the body of the patient if the control and analysis unit (10) determines with reference to the measured signals (23) recorded in response to the stimuli (40) applied in a time-offset manner in the first, second and third regions that the stimuli (40) applied in a time-offset manner do not reduce the pathologically synchronous and oscillatory activity of the stimulated neurons.

9. An apparatus (1) in accordance with claim 7 or claim 8, wherein the control and analysis unit (10) selects one or more further regions on the surface of the body of the patient after the application of the time-offset stimuli (40) in the first, second and third regions and controls the stimulation unit (11) such that it applies stimuli (40) in a time-offset manner in the first region, in the second region, in the third region, and in the further selected region or regions, said stimuli effecting a phase reset of the neuronal activity of the stimulated neurons.

10. An apparatus (1) in accordance with any one of the preceding claims, wherein the stimulation unit (11) has a plurality of stimulation elements (35) that are arranged in an array and that generate the mechanically tactile and/or thermal stimuli (22).

11. An apparatus (1) in accordance with claim 10, wherein an activation group comprises those stimulation elements (35) that are located at least partly within a specific shape that can be mapped on the array, with the shape moving over the array on a movement of the activation group and the stimulation elements (35) encompassed by the activation group changing accordingly.

12. An apparatus (1) in accordance with claim 11, wherein on the scanning of at least a part of the surface of the body of the patient by the stimulation unit (11), the activation group migrates along the path over the array and the stimulation elements (35) encompassed by the activation group generate the periodic stimuli.

13. An apparatus (1) in accordance with any one of the claims 10 to 12, wherein the stimulation elements (34) arranged in the array are fastened to a cuff (36) that is configured such that it can be fixed to the body of the patient.

14. An apparatus (1) in accordance with any one of the claims 11 to 13, comprising an input unit (37) that is configured such that a user can input the shape of the activation group and/or the path along which the activation group migrates into the input unit (37).

15. Software for execution in a data processing system in the apparatus of claim 1, wherein the software

- generates control signals for controlling a non-invasive stimulation unit (11), wherein the stimulation unit (11) applies mechanically tactile and/or thermal stimuli (22) to the surface of the body of a patient, wherein the stimuli (22) stimulate neurons having a pathologically synchronous and oscillatory neuronal activity;
- measured signals (23) are recorded which reproduce a neuronal activity of the stimulated neurons;
- the stimulation unit (11) scans at least a part of the surface of the body of the patient along a path and in so doing periodically applies stimuli;
- at least two regions on the surface of the body of the patient along the path are selected with reference to the measured signals recorded in response to the periodic application of the stimuli, with the phase synchronization between the periodic application of the stimuli and the neuronal activity of the stimulated neurons respectively having a local maximum in said regions; and
- the stimulation unit (11) applies stimuli (40) in a time-offset manner in the at least two selected regions, the stimuli effecting a phase reset of the neuronal activity of the stimulated neurons.

## Revendications

1. Un dispositif (1) de stimulation neuronale comprenant

- une unité de stimulation non invasive (11) pour appliquer des stimuli (22) mécaniques tactiles et/ou thermiques à la surface corporelle d'un patient, sachant que les stimuli (22) stimulent des neurones présentant une activité neuronale pathologiquement synchrone et oscillatoire,
- une unité de mesure (12) destinée à recevoir des signaux de mesure (23) représentant une activité neuronale des neurones stimulés, et
- une unité de commande et d'analyse (10) pour commander l'unité de stimulation (11) et pour analyser les signaux de mesure (23), sachant que l'unité de commande et d'analyse (10) est conçue de façon qu'elle :
- entraîne l'unité de stimulation (11) de manière que celle-ci balaye au moins une partie de la surface corporelle du patient le long d'un trajet et applique périodiquement des stimuli,
- sélectionne, sur la base des signaux de mesure enregistrés en réponse à l'application périodique des stimuli, au moins deux zones de la surface corporelle du patient le long de la voie, dans lesquelles la synchronisation de phase entre l'application périodique de stimuli et l'activité neuronale des neurones stimulés présente respectivement un maximum local, et qu'elle
- entraîne l'unité de stimulation (11) de telle manière que celle-ci applique avec un décalage temporel des stimuli (40) dans les au moins deux zones sélectionnées, qui provoquent une remise à zéro de phase de l'activité neuronale des neurones stimulés.

2. Le dispositif (1) d'après la revendication 1, sachant que l'unité de commande et d'analyse (10) vérifie si les stimuli (40) appliqués avec un décalage temporel réduisent l'activité pathologiquement synchrone et oscillatoire des neurones stimulés, sur la base des signaux de mesure (23) enregistrés en réponse aux stimuli (40) appliqués avec un décalage temporel.

3. Le dispositif (1) d'après la revendication 1 ou 2, sachant que les au moins deux zones sélectionnées par l'unité de commande et d'analyse (10) en réponse à l'application périodique des stimuli comprennent une première zone et une deuxième zone, et sachant que l'unité de commande et d'analyse (10) déplace la deuxième zone sur la surface corporelle du patient, si l'unité de commande et d'analyse (10) détermine, sur la base des signaux de mesure (23) enregistrés en réponse aux stimuli (40) appliqués avec un décalage temporel, que les stimuli (40) appliqués avec un décalage temporel ne réduisent pas l'activité pathologiquement synchrone et oscillatoire des neurones stimulés.

**4.** Le dispositif (1) d'après la revendication 3, sachant que l'unité de commande et d'analyse (10) déplace la deuxième zone de la surface corporelle du patient jusqu'à ce que l'unité de commande et d'analyse (10) détermine, sur la base des signaux de mesure (23) enregistrés en réponse aux stimuli (40) appliqués avec un décalage temporel, que les stimuli (40) appliqués avec un décalage temporel réduisent l'activité pathologiquement synchrone et oscillatoire des neurones stimulés.

**5.** Le dispositif (1) d'après la revendication 3 ou 4, sachant que l'unité de commande et d'analyse (10) sélectionne, après l'application des stimuli (40) avec un décalage temporel dans les zones première et deuxième, une troisième zone sur la surface corporelle du patient, et entraîne l'unité de stimulation (11) de manière que celle-ci applique des stimuli (40) avec un décalage temporel dans la deuxième zone et la troisième zone mais pas dans la première zone, qui provoquent une remise à zéro de phase de l'activité neuronale des neurones stimulés.

**6.** Le dispositif (1) d'après la revendication 5, sachant que l'unité de commande et d'analyse (10) déplace la troisième zone sur la surface corporelle du patient si l'unité de commande et d'analyse (10) détermine, sur la base des signaux de mesure (23) enregistrés en réponse aux stimuli (40) appliqués avec un décalage temporel dans les zones deuxième et troisième, que les stimuli (40) appliqués avec un décalage temporel ne réduisent pas l'activité pathologiquement synchrone et oscillatoire des neurones stimulés, et/ou sachant que l'unité de commande et d'analyse (10) sélectionne, après l'application des stimuli (40) décalés dans le temps dans les zones deuxième et troisième, une ou plusieurs autres zones sur la surface corporelle du patient, et qu'elle entraîne l'unité de stimulation (11) de telle manière que celle-ci applique avec un décalage temporel dans deux des zones sélectionnées mais pas dans les autres zones des stimuli (40), qui provoquent une remise à zéro de phase de l'activité neuronale des neurones stimulés.

**7.** Le dispositif (1) d'après la revendication 3 ou 4, sachant que l'unité de commande et d'analyse (10) sélectionne, après l'application des stimuli (40) décalés dans le temps dans les zones première et deuxième, une troisième zone sur la surface corporelle du patient, et qu'elle entraîne l'unité de stimulation (11) de manière que celle-ci applique avec un décalage temporel des stimuli (40) dans la première zone, la deuxième zone et la troisième zone, qui provoquent une remise à zéro de phase de l'activité neuronale des neurones stimulés.

**8.** Le dispositif (1) d'après la revendication 7, sachant que l'unité de commande et d'analyse (10) déplace la troisième zone sur la surface corporelle du patient si l'unité de commande et d'analyse (10) détermine, sur la base des signaux de mesure (23) enregistrés en réponse aux stimuli (40) appliqués avec un décalage temporel dans les zones première, deuxième et troisième, que les stimuli (40) appliqués avec un décalage temporel ne réduisent pas l'activité pathologiquement synchrone et oscillatoire des neurones stimulés.

**9.** Le dispositif (1) d'après la revendication 7 ou 8, sachant que l'unité de commande et d'analyse (10) sélectionne, après l'application avec un décalage temporel des stimuli (40) dans les zones première, deuxième et troisième une ou plusieurs autres zones sur la surface corporelle du patient, et qu'elle entraîne l'unité de stimulation (11) de manière que celle-ci applique avec un décalage temporel des stimuli (40) dans la première zone, la deuxième zone, la troisième zone et la ou les autres zones sélectionnées, qui provoquent une remise à zéro de phase de l'activité neuronale des neurones stimulés.

**10.** Le dispositif (1) d'après une des revendications précédentes, sachant que l'unité de stimulation (11) présente une pluralité d'éléments de stimulation (35) disposés en réseau, qui génèrent les stimuli (22) mécaniques tactiles et/ou thermiques.

**11.** Le dispositif (1) d'après la revendication 10, sachant qu'un groupe d'activation comprend les éléments de stimulation (35) qui se trouvent au moins partiellement à l'intérieur d'une forme spécifique pouvant être représentée sur le réseau, sachant que, lors d'un mouvement du groupe d'activation, la forme se déplace à travers le réseau et que les éléments de stimulation (35) inclus dans le groupe d'activation changent en conséquence.

**12.** Le dispositif (1) d'après la revendication 11, sachant que, lorsqu'au moins une partie de la surface corporelle du patient est balayée par l'unité de stimulation (11), le groupe d'activation se déplace le long du trajet à travers le réseau, et que les éléments de stimulation (35) inclus dans le groupe d'activation génèrent les stimuli périodiques.

**13.** Le dispositif (1) d'après une des revendications de 10 à 12, sachant que les éléments de stimulation (35) disposés dans le réseau sont fixés à un brassard (36) qui est réalisée de manière à pouvoir être fixé au corps du patient.

**14.** Le dispositif (1) d'après une des revendications de 11 à 13 comprenant une unité d'entrée (37) réalisée de manière qu'un utilisateur peut entrer dans l'unité d'entrée (37) la forme du groupe d'activation et/ou le chemin le long duquel le groupe d'activation se déplace.

**15.** Un logiciel à exécuter dans un système de traitement de données dans le dispositif d'après la revendication 1, sachant que
le logiciel

- génère des signaux de commande pour l'entraînement d'une unité de stimulation non invasive (11), l'unité de stimulation (11) appliquant mécaniquement des stimuli tactiles et/ou thermiques (22) à la surface corporelle d'un patient, sachant que les stimuli (22) stimulent des neurones qui présentent une activité neuronale pathologiquement synchrone et oscillatoire,
- des signaux de mesure (23) étant reçus qui reflètent une activité neuronale des neurones stimulés,
- l'unité de stimulation (11) balaye au moins une partie de la surface corporelle du patient le long d'un trajet et applique périodiquement des stimuli,
- sur la base des signaux de mesure enregistrés en réponse à l'application périodique des stimuli, au moins deux zones de la surface corporelle du patient le long du trajet sont sélectionnées, dans lesquelles la synchronisation de phase entre l'application périodique des stimuli et l'activité neuronale des neurones stimulés présente chacune un maximum local, et
- l'unité de stimulation (11) applique des stimuli (40) avec un décalage temporel dans les au moins deux zones sélectionnées, qui provoquent une remise à zéro de phase de l'activité neuronale des neurones stimulés.

<u>Fig.1</u>

## Fig.2

Selektion wirksamer Stimulationsorte
mittels Entrainment - Test

Selektion wirksamer Paare
mittels CR - Test

Selektion wirksamer Gruppen
mittels CR - Test

## Fig.3

## Fig.4

11

35

36

$x_1$ $x_2$ $x_3$   $x_{k-2}$ $x_k$
$x_{k-1}$ $x_{k+1}$   $x_M$

## Fig.5

P

$T_{stim}$   D

t

## Fig.6

I

$T_{vib}$

$I_{max}$

t

## Fig.7

## Fig.8

$T_{stim}$

40  40  40

Aktivierungsgruppe 1

t

$T_{stim}/2$  $T_{stim}$  40  40

40  40

Aktivierungsgruppe 2

t

## Fig.9

$T_{stim}$

40  40  40

40

Aktivierungsgruppe 1

t

$T_{stim}/4$  $T_{stim}$  40

40  40  40

Aktivierungsgruppe 2

t

$T_{stim}/2$  $T_{stim}$  40

40  40  40

Aktivierungsgruppe 3

t

$3T_{stim}/4$  $T_{stim}$  40

40  40  40

Aktivierungsgruppe 4

t

Fig.10

Fig.11

Fig.12

Fig.13

Fig.14

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013117655 A1

**In der Beschreibung aufgeführte Nicht-Patentliteratur**

- Phase Synchronization: From Theory to Data Analysis. **B. M. G. ROSENBLUM ; A. S. PIKOVSKY ; C. SCHÄFER ; J. KURTHS ; P. A. TASS.** Handbook of Biological Physics. Elsevier, 2000
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345
- **P. TASS ; M.G. ROSENBLUM ; J. WEULE ; J. KURTHS ; A. PIKOVSKY ; J. VOLKMANN ; A. SCHNITZLER ; H.-J. FREUND.** Detection of n:m Phase Locking from Noisy Data: Application to Magnetoencephalography. *Phys. Rev. Lett.,* 1998, vol. 81 (15), 3291-3294
- Phase Synchronization: From Theory to Data Analysis. **M. G. ROSENBLUM ; A. S. PIKOVSKY ; C. SCHÄFER ; J. KURTHS ; P. A. TASS.** Handbook of Biological Physics. Elsevier, 2000
- **E. BATSCHELET.** Circular Statistics in Biology. Academic Press, 1981
- **N. H. KUIPER.** Tests concerning random points in a circle. *Proc. K. Ned. Akad. Wet., Ser. A: Math. Sci.,* 1960, vol. 63, 38

- Transmission of stimulus-locked responses in two coupled phase oscillators. **P. A. TASS.** Phys. Rev. E. 2004, vol. 69, 051909-1, 24
- **P. LANDA.** Nonlinear Oscillations and Waves in Dyanmical Systems. Kluwer Academic Publishers, 1996
- **P. A. TASS.** Transmission of stimulus-locked responses in two coupled phase oscillators. *Phys. Rev. E,* 2004, vol. 69, 051909-1, 24
- **P. A. TASS.** Stochastic phase resetting of two coupled phase oscillators stimulated at different times. *Physical Review E,* 2003, vol. 67, 051902-1, 051902-15
- **N. E. HUANG et al.** The empirical mode decomposition and the Hilbert spectrum for nonlinear and non-stationary time series analysis. *Proceedings of the Royal Society of London Series A,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345
- **P. A. TASS.** *Stochastic phase resetting of two coupled phase oscillators stimulated at different times*
- **P. A. TASS.** Stochastic phase resetting of two coupled phase oscillators stimulated at different times. *Phys. Rev. E,* 2003, vol. 67, 051902-1, 051902-15